# EUROPEAN PATENT SPECIFICATION

(11) **EP 1 310 224 B1**
(45) Date of publication and mention of the grant of the patent: **20.08.2008**
(21) Application number: 02257633.4
(22) Date of filing: 05.11.2002
(51) Int. Cl.: A61F 13/15

(54) **Absorbent product**
Absorbierender Artikel
Article absorbant

(30) Priority: 07.11.2001 JP 2001342184
(43) Date of publication of application: 14.05.2003
(73) Proprietor: Koyo Disposable Goods Co., Ltd., Yokohama-shi, Kanagawa 247-0014 (JP)
(72) Inventor: Takagi, Katsumasa, Yokohama-shi, Kanagawa (JP); Terada, Sadayoshi, Kamakura-shi, Kanagawa (JP); Yamazaki, Rie, Hiratsuka-shi, Kanagawa (JP)
(74) Representative: Jehan, Robert

(56) References cited:
- EP-A- 0 321 732
- WO-A-01/13852
- WO-A-91/04724
- US-A- 4 081 301
- US-A- 4 430 088
- US-A- 4 795 451
- US-A- 5 259 902
- US-A- 5 660 657
- US-A- 5 876 390
- US-A- 5 971 970

## Description

The present invention relates to an absorbent product such as an absorbent core of a disposable diaper and absorbent product excellent in fitting property and shape retention property, in the preferred embodiment for a disposable diaper; including a method of manufacturing the absorbent product.

US 4,795,451 discloses a disposable absorbent pad for use as a baby diaper or an adult incontinent brief wherein the backing sheet of the diaper has a pair of continuous bands of adhesive arranged in a stepped generally longitudinal configuration thereon. An elastic band is disposed over the mid-portion of the adhesive being stepped to the side, out of contact with the elastic. When the pads are cut in the assembly process, only that portion of elastic in contact with the adhesive causes the pad to gather. The remaining end portions of the adhesive bands are secured to the intermediate layer of absorbent fluff material, helping it remain in place, and act as a fluid barrier on the sides of the absorbent pad.

US 4,081,301 discloses a method in which a continuous elastic ribbon is fed to a diaper web assembly station in a stretched condition. While travelling to the assembly station, an adhesive is applied to discrete lengths of the elastic ribbon at regularly spaced intervals. Simultaneously, discrete absorbent core segments as well as webs of moisture-impervious backsheet material and moisture-pervious topsheet material are fed to the diaper web assembly station. At the station, the stretched elastic ribbon is adhered to the moisture-impervious backsheet web in the discrete areas of the elastic which are covered by adhesive at predetermined points along the length of said web. After the adhesive has set up, the assembled web and the elastic contained therein is severed in its unadhered areas, whereupon the unadhered end portions become relaxed and inactive without affecting the functionality of the adhered portions in the ultimate assemblage.

US 5,259,902 provides a method for continuously attaching discrete lengths of a tensioned elastic material to predetermined, isolated portions of moving absorbent articles, said absorbent articles having a topsheet, a backsheet, and an absorbent core. A continuous web of backsheet material is fed to an assembly station. An absorbent core is secured to said continuous web of backsheet material. Intermittent portions of a continuous ribbon of elastic material are secured to said backsheet at predetermined discrete locations. A ribbon of elastic material is cut at predetermined locations. The backsheet and the stretched elastic are maintained in a substantially tensioned state. A continuous web of topsheet material is fed to said assembly station. The topsheet web is secured to said backsheet web. The absorbent articles is maintained in a substantially tensioned state. Discrete portions of stiffening materials are adhered to said continuous web of wrapper material. A panty fastening adhesive is applied to said continuous web of wrapper material. The absorbent articles are fed onto said continuous web of wrapper material. The absorbent articles are adhered to said wrapper material. The wrapper maintains said absorbent articles in a substantially tensioned state.

The main function of an absorbent product used in disposable diapers for babies and adults is to absorb and contain body wastes discharged due to incontinence and the like. However, the body wastes may leak from a gap between the absorbent product and the leg or the waist of a wearer without being absorbed in the absorbent product. Absorbent products such as disposable diapers and sanitary napkins which have been on the market recently mainly comprise an inner surface sheet, which is composed of a liquid pervious non-woven fabric, positioned on a skin side when the absorbent product is applied to the human body, a liquid impervious back sheet, which is composed of polyethylene, positioned on an outer surface when the absorbent product is applied to the human body, and an absorbent core interposed between the sheets.

More specifically, standing cuff gather sections are formed of standing cuff gathers composed of a non-woven fabric on both sides of a disposable diaper and an elastic material along the inner edge of each standing cuff gather. Side flap portions in which the absorbent core does not exist are formed of liquid impervious back sheet portions extending outward in a longitudinal direction from the side edges of the absorbent core and the external sheet portions of the standing cuff gathers, and further a number of elastic rubber strings are disposed between the standing cuff gathers and the side flap portions along the longitudinal direction of the disposable diaper; thereby pleated flat gathers are formed along the side flap portions.

That is, the disposable diaper is provided with leg elastic gathers for improving fitting property as well as for sealing around legs to prevent leakage when it is applied to the human body, waist gathers for sealing around the waist, and further barrier cuffs (Japanese Examined Patent Publication No. 6-93901, Patent No. 2561675, which corresponds to US 4,695,278) called standing cuff gathers which are effective with soft faeces and disposed inside of the leg gathers in a longitudinal direction on both ends of an absorbent core.

In the conventional disposable diaper as described above, the fitting property and leakage preventing property are improved by the leg elastic gathers and the barrier cuffs, However, the conventional disposable diaper is disadvantageous in that soft faeces and urine are attached to and absorbed by an absorbent core, and when the amount of the absorbed substances increases as time passes, the absorbent core is dislocated downward by the weight of the substances and a gap is formed in the crotch portion. Thus, a problem arises in that the sealing property of the leg gathers and the barrier cuffs is made insufficient, and the substances leak and emit a bad smell from the gap.

The present invention seeks to provide an improved absorbent product.

According to an aspect of the present invention, there is provided an absorbent product as specified in claim 1.

According to another aspect of the present invention, there is provided a method of manufacturing an absorbent product as specified in claim 9.

The preferred embodiment can provide a disposable diaper excellent in leakage prevention and capable of giving a worry-free feeling to a wearer due not only to the improved fitting property of the disposable diaper when it is applied to the human body but also to the shape retention property thereof even if the amount of substances absorbed increases.

More specifically, the preferred embodiments provide (with reference to the accompanying drawings) an absorbent product such as pad type and flat sheet type disposable diapers as well as a sanitary napkin and similar goods that consist of fluffed pulp, synthetic fibres, super absorbent material, and the like, wherein the absorbent product includes a liquid pervious inner surface sheet 1, a liquid impervious back sheet 2 positioned on an outside surface, an absorbent core 3 interposed between the inner surface sheet 1 and the back sheet 2, and elastic materials 4, typically composed of a number of elastic strings or strands in a linear shape, disposed adjacent to both sides of the absorbent core so as to longitudinally traverse the absorbent core extending on a human body from the back to the stomach through the crotch portion when the absorbent product is applied to the human body, wherein the expansion ratio of the elastic materials is sequentially increased laterally from the centre to the external portions thereof.

When the absorbent product such as the pad type and flat sheet type disposable diaper as well as the sanitary napkin and similar goods is applied to the human body from the back to the stomach through the crotch portion, the liquid pervious inner surface sheet 1 faces the skin side, the liquid impervious back sheet 2 faces the outside surface thereof, and the absorbent core 3 interposed between the sheets 1 and 2 is held by the elastic materials 4.

Accordingly, when urine, faeces, body fluids, and the like are discharged, even if the weight of the absorbent core is increased by absorbing the substances, the leakage of the substances can be prevented because the shape retention property of the absorbent product is maintained by the tension of the elastic materials 4 longitudinally traversing the absorbent body 3.

Preferably, fastening materials 5', 5' are attached to both end portions of the absorbent product, and the absorbent product is fastened to a waist belt holder 7 and to tape type diapers 10L, 10M, and 10N, to pants type diapers 10P, 10Q, and 10R and to a diaper extend pants through the fastening materials 5', 5' such that the absorbent product extends from the back to the stomach through the crotch portion when it is applied to the human body.

Advantageously, the absorbent product is fastened to the waist belt holder as well as to the tape type diapers 10L, 10M, and 10N, to the pants type diapers 10P, 10Q, and 10R and to the diaper extend pants through the fastening materials such that the absorbent product extends from the back to the stomach through the crotch portion when it is applied to the human body.

Accordingly, not only can a secure fit be obtained around the waist when the absorbent product is applied to the human body but also the absorbent product can be replaced when it becomes dirty because it is fastened with the fastening materials.

In another embodiment, there is provided an absorbent product which includes wing type disposable diapers 10C, 10D, and 10E and T-type disposable diapers 10F, 10G, and 10H as well as sanitary napkins 10A provided with a pants or a belt holder, and similar goods, each including the absorbent product.

When each of the wing type disposable diapers 10C, 10D, and 10E and the T-type disposable diapers 10F, 10G, and 10H as well as the sanitary napkin 10A is applied to the human body, the absorbent core 3 facing the skin is held by the elastic strings 4 that are disposed adjacent to both sides of the absorbent core 3 so as to longitudinally traverse the human body from the back to the stomach through the crotch portion.

Accordingly, when urine, faeces, and body fluids are discharged, even if the weight of the absorbing body 3 facing the skin is increased by absorbing the substances, the leakage of the substances can be prevented because the formation of a gap in the crotch portion is prevented by the tension of the elastic strings 4 longitudinally traversing the absorbing body 3, and the shape retention properties of the disposable diapers 10C, 10D, 10E, 10F, 10G, and 10H as well as the sanitary napkin 10A are maintained.

Preferably, when provided as disposable diapers, wherein the tape type disposable diapers 10L, 10M, and 10N are diapers each including an absorbent product and formed of fluffed pulp, synthetic fibres, a super absorbent material, and the like, and each of the tape type disposable diapers 10L, 10M and 10N include a liquid pervious inner surface sheet 1 positioned on the skin side when the disposable diaper is applied to the human body, a liquid impervious back sheet 2 positioned on the outside when the disposable diaper is applied to the human body, a waist hole section and leg hole sections, an absorbent core 3 interposed between the inner surface sheet 1 and the back sheet 2, and elastic materials 4 directly attached to the back sheet 2 extending from the back to the stomach through the crotch portion when the disposable diaper is applied to the human body so as to longitudinally traverse the absorbent core 3.

When each of the tape type disposable diapers 10L, 10M, and 10N is applied to the human body, the absorbent core 3 of the absorbent product facing the skin is held by the elastic materials 4 adjacent to both sides of the absorbent core so as to longitudinally traverse the human body from the back to the stomach through the crotch portion.

Accordingly, when urine, faeces, and body fluids are discharged, even if the weight of the absorbing body 3 facing the skin is increased by absorbing the substances, the leakage of the substances can be prevented because the formation of a gap in the crotch portion is prevented by the tension of the elastic materials 4 longitudinally traversing the absorbing body 3 and the shape retention property of the tape type disposable diapers 10L, 10M, and 10N is maintained.

In embodiments providing pants-type disposable diapers 10P, 10Q, and 10R these are diapers each including an absorbent product consisting of fluffed pulp, synthetic fibres, a super absorbent material, and the like, and each of the pants disposable diapers, which has an outside surface, an inside surface, a front portion, a rear portion and a crotch portion including a liquid pervious inner surface sheet 1, a liquid impervious back sheet 2 on the inner surface sheet, an absorbent core 3 interposed between the inner surface sheet 1 and the back sheet 2, and elastic materials 4 directly attached between the inner surface sheet 1 and the back sheet 2, which extends from the back to the stomach through the crotch portion when the disposable diaper is applied to the human body, and elastic materials directly attached inside of the inner surface sheet 1, so as to longitudinally traverse the absorbent core 3.

When each of the pants type disposable diapers 10P, 10Q, and 10R, each having the absorbent product fastened to the inner surface sheet 1, is applied to the human body, the absorbent core 3 of the absorbent product facing the skin is held by the elastic materials disposed adjacent to both sides of the absorbent core 3 so as to longitudinally traverse the human body from the back to the stomach through the crotch portion.

Accordingly, when urine, faeces, and body fluids are discharged, even if the weight of the absorbent core 3 facing the skin is increased by absorbing the substances, the leakage of the substances can be prevented because the formation of a gap in the crotch portion is prevented by the tension of the elastic materials 4 longitudinally traversing the absorbent core 3, and the shape retention property of the pants type disposable diapers 10P, 10Q, and 10R is maintained.

Advantageously, the absorbent core 3 of the disposable diaper as well as the elastic materials 4 of the absorbent product are symmetrically disposed in a direction where they spread downward from an upper end portion or to the upper end portion from below at an inclining angle of 0° to 45° to the centre line of the absorbent core 3.

When such a disposable diaper is applied to the human body, the absorbent core 3 as well as the elastic materials 4 of the absorbent product are symmetrically disposed in a direction where they spread downward from an upper end portion or to the upper end portion from below at an inclining angle of 0° to 45° to the centre line of the absorbent core 3.

Accordingly, when urine, faeces, body fluids, and the like are discharged, even if the weight of the absorbent core 3 is increased by absorbing these substances, the leakage of the substances can be prevented because the shape retention property of the disposable diaper is maintained by the elastic force of the elastic materials 4 obliquely traversing the absorbent core 3 with respect to the centre line thereof.

Preferably, the expansion ratio of the elastic materials 4 is set larger on both side ends of the absorbent core 3 than the expansion ratio of the centre line thereof. Thus, when the absorbent product is applied to the human body from the back to the stomach through the crotch portion, the absorbent core 3, which is interposed between the liquid pervious inner surface sheet facing the skin and the liquid impervious back sheet 2 extending the outer surface of the inner surface sheet 1, is pressed against the skin strongly on the external portions thereof and softly on the internal portion thereof by the difference of the expansion ratio of the elastic materials 4, forming an approximate arc shape having a gap in the inside thereof.

Accordingly, the elastic materials disposed on both external portions of the absorbent product are pressed against the skin more strongly as compared with the internal portion thereof when the absorbent product is applied to the human body. Thus, when urine, faeces, body fluids, and the like are discharged, even if the weight of the absorbent core 3 is increased by absorbing the substances, the leakage of the substances can be prevented by the elastic force of the elastic materials 4.

One embodiment provides a disposable diaper including standing cuff gathers on both sides of the absorbent core 3 and having elastic materials 7, 8 at the standing extreme ends thereof, wherein the elastic materials 7, 8 of the standing cuff gathers have an expansion ratio larger than that of the elastic materials 4 of the absorbent core 3 so that the contraction coefficient of the elastic strings gradually increases from the longitudinal centre line of the absorbent product to reach maximum at the both sides thereof.

Thus, when the disposable diaper, which includes the standing cuff gathers on both sides of the absorbent core 3 and having the elastic materials 7, 8 at the standing extreme ends thereof, is applied to the human body from the back to the stomach through the crotch portion, the extreme ends of the standing cuff gathers facing the skin are strongly pressed against the skin at an expansion ratio larger than that of the elastic materials 4 of the absorbent core 3.

Accordingly, the elastic materials 4 of the extreme end of the standing cuff gathers on both sides of the absorbent core 3 when the disposable diaper is applied to the human body press the skin more strongly than the absorbent core 3 disposed internally. Thus, when urine, faeces, body fluids, and the like are discharged, even if the weight of the absorbent core 3 is increased by absorbing the substances, the leakage of the substances can be prevented by the large expansion ratio of the elastic materials 4 provided with the standing cuff gathers.The preferred method of manufacturing an absorbent product includes the steps of placing a super absorbent material 64 mixed in an air stream on a tissue web where a continuous web is formed just before fluff pulp 31 as a material of an absorbent core is mixed with air and blown onto a suction drum 35, holding the tissue web on the upper surface thereof by a tissue folding device 65 in a process in which the tissue web is transported by a conveyer, introducing the tissue web to press rolls 39 through belt presses 38, forming a continuous absorbent core 3 by pressing the tissue web to a predetermined density by the press rolls, cutting off the absorbent core 3 by a mat cutter through an emboss roll press 40, joining a liquid pervious inner surface sheet and a liquid impervious back sheet 43 having binders 46,47 previously applied to one of the upper and lower surfaces thereof to the cut-off absorbent core 3, and supplying a non-woven fabric sheet, to which a binder is applied, to the back sheet 43 that is attached to the lower surface of the absorbent core 3 together with a number of string-like elastic materials 44 such that the elastic materials are sandwiched between the absorbent core 3 and the non-woven fabric sheet 45 and disposed adjacent to both sides of the absorbent core in a state in which the elastic materials 44 are applied with tension such that the expansion ratio of the elastic materials 44 is sequentially increased from the centre to the external portions thereof in a latitudinal (width) direction and attached between the absorbent core 3 and the back sheet 43, or between the back sheet 43 and the non-woven fabric sheet 45.

Accordingly, the elastic materials 44 are sandwiched and adhered between the absorbent core 3 and the back sheet 43, or between the back sheet 43 and the non-woven fabric sheet 45 while being applied with tension, sequentially increasing the expansion ratio thereof from the centre to the external portions thereof laterally in the process of continuously forming the absorbent product. Thus, the absorbent product, which is curved in an approximate arc shape such that the expansion ratio thereof is sequentially increased laterally from the centre to the external portions thereof, can be continuously manufactured effectively.

Embodiments of the present invention are described below, by way of example only, with reference to the accompanying drawings, in which:
Figure 1 is a plan view of a pad type (longitudinal) absorbent product according to a first embodiment of the present invention;
Figure 2 is a plan view of a pad type (gourd type) absorbent product according to the first embodiment of the present invention;
Figure 3 is a plan view of a pad type (elliptical) absorbent product according to the first embodiment of the present invention;
Figure 4 is a plan view of a pad type (elliptical) absorbent product according to the first embodiment of the present invention;
Figure 5 is a plan view of a flat sheet type absorbent product according to the first embodiment of the present invention;
Figures 6A and 6B are plan views of flat sheet type absorbent products according to the first embodiment of the present invention;
Figures 7A and 7B are plan views of sanitary napkins according to the first embodiment of the present invention;
Figure 8A is a plan view of a sanitary napkin according to the present invention, and Figure 8B is a sectional view of the sanitary napkin taken along the line B-B of Figure 8A;
Figures 9A to 9E are sectional views taken along the lines A-A of Figures 1 to 8A and show the disposition of elastic materials to an absorbent core of the absorbent product according to the first embodiment of the present invention;
Figure 10A is a side sectional view of a pad type disposable diaper according to a second embodiment of the present invention having an absorbent product attached to a waist belt holder with a fastening means, and Figure 10B is a developed plan view of the absorbent product;
Figure 11A is a plan view of a tape type disposable diaper according to the second embodiment of the present invention to which an absorbent product in a pad type disposable diaper is attached with fastening means, and Figure 11B is a sectional view of the tape type disposable diaper taken along the line C-C of Figure 11A;
Figure 12 is a side sectional view of a short type disposable diaper according to the second embodiment of the present invention fastened to a sanitary napkin with fastening means;
Figure 13 is a plan view of a wing type disposable diaper according to a third embodiment of the present invention;
Figure 14A is a plan view of a T-type disposable diaper, and Figure 14B is a sectional view of the T-type disposable diaper taken along the line E-E of Figure 14A;
Figure 15 is a plan view of a wing type disposable diaper according to the third embodiment of the present invention;
Figure 16 is a plan view of a wing type disposable diaper according to the third embodiment of the present invention to which an absorbent product is fastened likewise;
Figure 17 is a plan view of a T-type disposable diaper according to the third embodiment of the present invention to which an absorbent product is fastened likewise;
Figure 18 is a plan view of a T-type disposable diaper according to the third embodiment of the present invention to which an absorbent product is fastened likewise;
Figure 19 is a plan view of a tape type disposable diaper according to a fourth embodiment of the present invention;
Figure 20 is a sectional view of the tape type disposable diaper taken along the line F-F of Figure 19;
Figure 21 is a developed plan view of a pants type disposable diaper according to the fourth embodiment of the present invention;
Figure 22 is a sectional view of the pants type disposable diaper taken along the line G-G of Figure 21;
Figure 23 is a plan view of a tape type disposable diaper according to the fourth embodiment of the present invention;
Figure 24 is a plan view of a tape type disposable diaper according to the fourth embodiment of the present invention;
Figure 25 is a plan view of a pants type disposable diaper according to a fifth embodiment of the present invention;
Figure 26 is a plan view of a pants type disposable diaper according to the fifth embodiment of the present invention likewise;
Figure 27 is a flowchart of a manufacturing process according to a sixth embodiment of the present invention for manufacturing an absorbent product;
Figure 28 is a view of an example of an apparatus for supplying a sufficient number of elastic materials in the state in which tension is applied to an absorbent product in such a manner that the expansion ratio of the elastic materials sequentially increases from the centre outwardly to both sides in the manufacturing process of the absorbent product; and
Figure 29 is a view explaining another example of the apparatus for supplying the elastic material likewise.

Although there are described below six principal embodiments of the present invention many other embodiments are disclosed and furthermore the features of the various described embodiments can be combined with and exchanged between one embodiment and another.

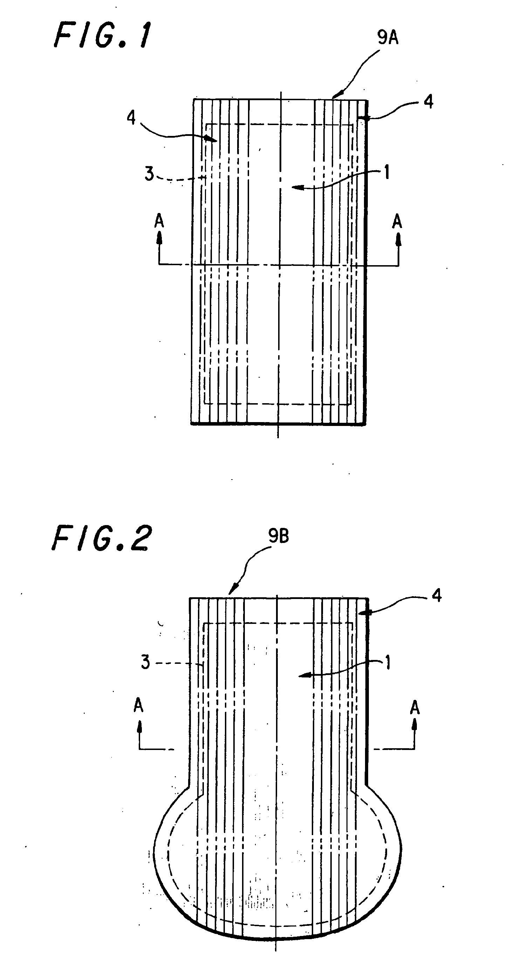

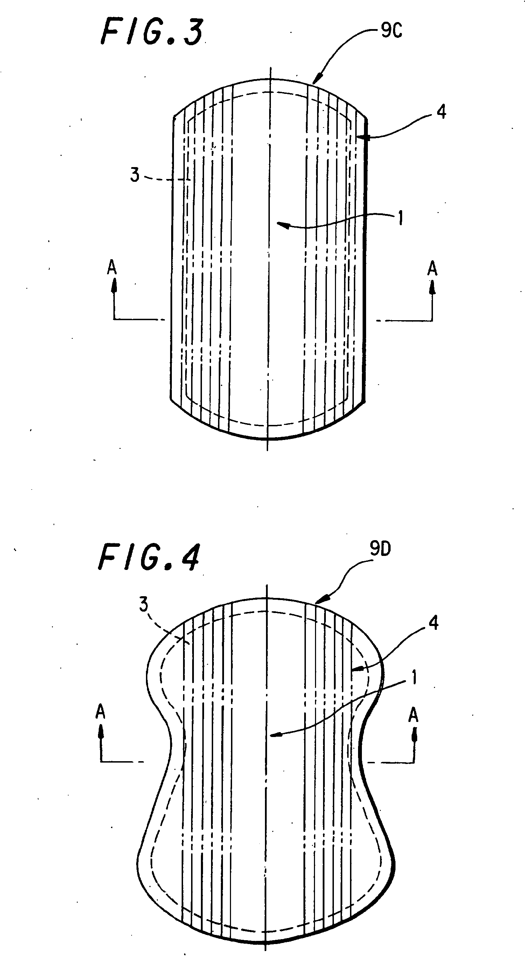

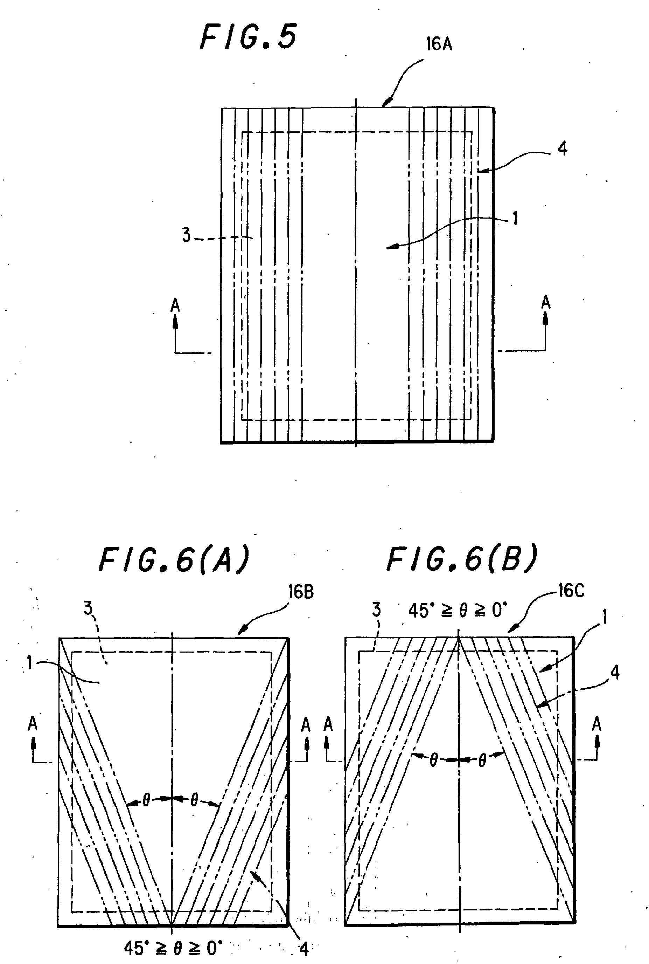

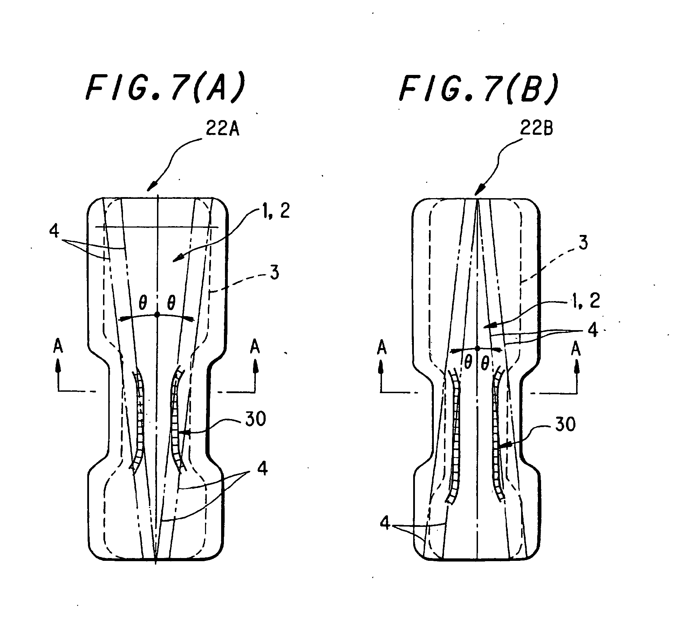

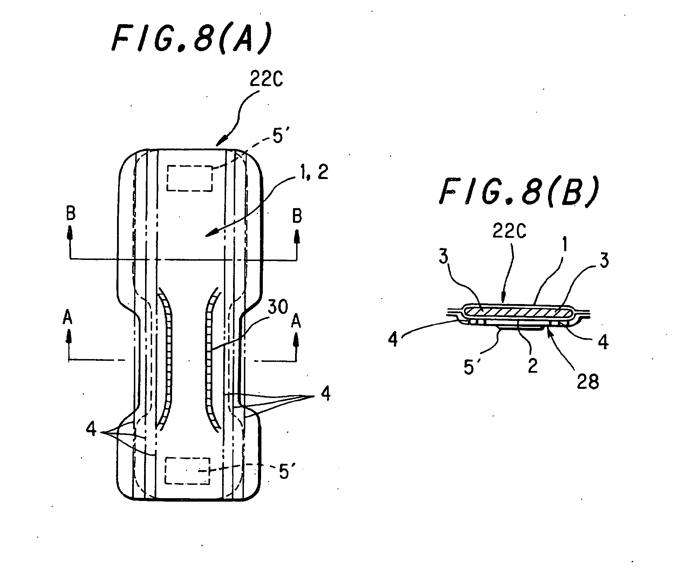

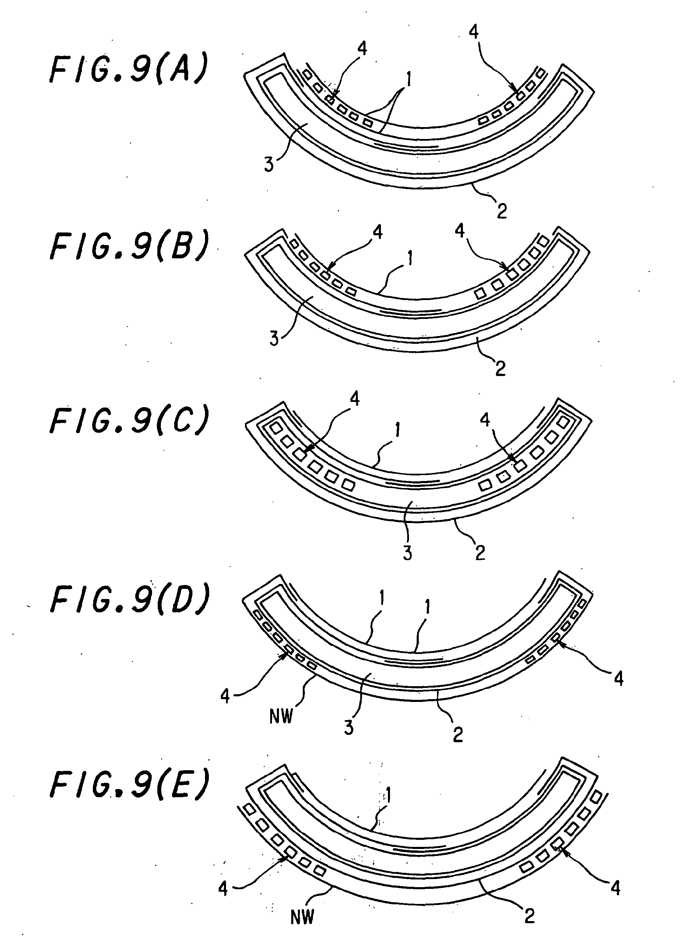

A first embodiment of the present invention is sequentially described in detail with respect to Figures 1 to 9 accompanied to the specification. Figure 1 is a plan view of a pad type (longitudinal) absorbent product according to a first embodiment of the present invention; Figure 2 is a plan view of a pad type (gourd type) absorbent product according to the first embodiment of the present invention; Figure 3 is a plan view of a pad type (elliptical) absorbent product according to the first embodiment of the present invention; Figure 4 is a plan view of a pad type (elliptical) absorbent product according to the first embodiment of the present invention; Figure 5 is a plan view of a flat sheet type absorbent product according to the first embodiment of the present invention; Figures 6A and 6B are plan views of flat sheet type absorbent products according to the first embodiment of the present invention; Figures 7A and 7B are plan views of sanitary napkins according to the first embodiment of the present invention; Figure 8A is a plan view of a sanitary napkin, and Figure 8B is a sectional view of the sanitary napkin taken along the line B-B of Figure 8A, and Figures 9A to 9E are sectional views taken along the lines A-A of Figures 1 to 8A and show the disposition of an elastic material to an absorbent core of the absorbent products according to the first embodiment of the present invention.

In Figure 1, reference numeral 9A denotes a longitudinal absorbent product according to the first embodiment of the present invention which relates to an absorbent product such as a longitudinal pad type disposable diaper, sanitary napkin, and similar products. The longitudinal absorbent product 9A is mainly formed of fluffed pulp, synthetic fibres, a super absorbent material, and the like in a longitudinal rectangular shape and composed of a liquid pervious inner surface sheet 1, a liquid impervious back sheet 2, a rectangular absorbent core 3, and elastic materials. The inner surface sheet 1 is composed of a non-woven fabric and positioned on the skin side when the absorbent product is applied to the human body; the back sheet 2 is composed of polyethylene and positioned on an outer surface side when the absorbent product is applied to the human body; the absorbent core 3 is composed of fluffed pulp and the like and interposed between sheets 1 and 2. Further, the elastic materials are composed of, for example, a number of elastic strings, elastic strands, flat rubbers, urethane films, and any other linear forms (elastic strings 4 are used in this embodiment). When the absorbent product is applied to the human body, the elastic materials are disposed adjacent to both sides of the absorbent core 3 so as to longitudinally traverse the absorbent core 3 which extends from the back to the stomach passing through the crotch.

Next, reference numeral 9B shown in Figure 2 denotes a gourd type absorbent product such as a gourd type pad type disposable diaper, sanitary napkin, and the like. The gourd type absorbent product 9B is formed of the same materials as those of the above absorbent product in the gourd shape and composed of a liquid pervious inner surface sheet 1, which is positioned on the skin side when the absorbent product is applied to the human body, a liquid impervious back sheet 2, which is positioned on the outer surface when the absorbent product is applied to the human body, a gourd type absorbent core 3 interposed between the sheets 1 and 2, and a number of elastic materials 4 disposed adjacent to both sides of the absorbent core 3.

Further, reference numeral 9C shown in Figure 3 denotes an elliptical absorbent product such as an elliptical pad type disposable diaper, sanitary napkin, and the like. The elliptical absorbent product 9C is formed of the same materials as those of the above absorbent products in a gourd shape and composed of a liquid pervious inner surface sheet 1, which is composed of a non-woven fabric and positioned on the skin side when the disposable diaper is applied to the human body, a liquid impervious back sheet 2, which is positioned on an outer surface when the absorbent product is applied to the human body, an elliptical absorbent core 3 interposed between the sheets 1 and 2, and a number of elastic strings 4 disposed adjacent to both sides of the absorbent core 3. Further, reference numeral 9D shown in Figure 4 denotes a modified elliptical absorbent product arranged in the same mode as the above absorbent products.

Next, reference numeral 16A shown in Figure 5 denotes a flat sheet type absorbent product such as a flat sheet type disposable diaper, sanitary napkin, and the like. The flat sheet type absorbent product 16A is formed of the same materials as those of the above absorbent products in the flat sheet shape and composed of a liquid pervious inner surface sheet 1, which is composed of a non-woven fabric and positioned on the skin side when the disposable diaper is applied to the human body, a liquid impervious back sheet 2, which is positioned on the outer surface when the absorbent product is applied to the human body, a flat-sheet-shaped absorbent core 3 interposed between sheets 1 and 2, and a quantity of elastic materials 4 disposed adjacent to both sides of the absorbent core 3.

Reference numeral 16B shown in Figure 6A denotes a flat sheet type absorbent product such as a flat sheet type disposable diaper, sanitary napkin, and the like. The flat sheet type absorbent product 16B is also arranged in the same mode as the above flat sheet type absorbent product 16A, and a number of elastic strings 4 disposed adjacent to both sides of an absorbent core 3 are attached symmetrically in such a direction that they spread from the lower end portion toward the upper end portion at an inclining angle of 45° ≥ θ ≥ 0° to the longitudinal centre line of the absorbent core 3.

Reference numeral 16C shown in Figure 6B denotes the flat sheet type absorbent product such as a flat sheet type disposable diaper, sanitary napkin, and the like. The flat sheet type absorbent product 16C is also arranged in the same mode as the above flat sheet type absorbent product 16B. However, the absorbent product 16C is different from the absorbent product 16B in that a number of elastic strings 4 disposed adjacent to both sides of an absorbent core 3 are attached symmetrically in such a direction that they spread from the upper end portion toward the lower end portion at an inclining angle of 45° ≥ θ ≥ 0° to the longitudinal centre line of the absorbent core 3.

Next, reference numerals 22A and 22B shown in Figures 7A and 7B denote sanitary napkins acting as absorbent products. As shown in Figure 7A, the sanitary napkin 22A is arranged in the same mode as the above absorbent products and composed of the same materials as those of the absorbent products. The sanitary napkin 22A has concave embossed portions 30 formed in correspondence to the crotch portion of an absorbent core 3 which extends from the back to the stomach passing though the crotch when the sanitary napkin 22A is applied to the human body. Further, a number of elastic materials 4 disposed adjacent to both sides of the absorbent core 3 are attached symmetrically in such a direction that they spread from the lower end portion toward the upper end portion at an inclining angle of 45° ≥ θ ≥ 0° to the longitudinal centre line of the absorbent core 3.

While the sanitary napkin 22B shown in Figure 7B is also arranged in the same mode as the sanitary napkin 22A, the former napkin is different from the latter napkin in that a number of elastic materials 4 are attached symmetrically in such a direction that they spread from the upper end portion toward the lower end portion at an inclining angle of 45° ≥ θ ≥ 0° to the longitudinal centre line of the absorbent core 3.

Further, the sanitary napkin 22C shown in Figures 8A and 8B is arranged in the same mode as the sanitary napkins 22A and 22B. The sanitary napkin 22C has a quantity of elastic materials 4 on the back surface of a liquid impervious back sheet 2 positioned on the outer surface when the sanitary napkin 22C is applied to the human body, the elastic strings 4 being disposed parallel with each other adjacent to both sides of an absorbent core 3 while longitudinally traversing the absorbent core 3 that extends, when the sanitary napkin 22C is applied to the human body, from back to stomach passing through the crotch, and non-woven fabrics 28 are bonded to the external portions of the elastic materials 4. Then, fasteners, 5', acting as adhering members are attached to the upper and lower ends of the non-woven fabrics 28.

Thus, according to the first embodiment of the present invention, when each of the longitudinal absorbent product 9A, the gourd type absorbent product 9B, the elliptical absorbent product 9C, the modified elliptical absorbent product 9D, the flat sheet type absorbent products 16A, 16B, and 16C, and the sanitary napkins 22A, 22B, and 22C each arranged in the pad type is applied to the human body from the back to the stomach through the crotch, the liquid pervious inner surface sheet 1 faces the skin and the outer surface of the inner surface sheet 1 is extended with the liquid impervious back sheet 2; and the absorbent core 3 interposed between both the sheets 1 and 2 is held by the elastic materials 4. Accordingly, when urine, faeces, and body fluids are discharged, even if the weight of the absorbent core 3 is increased by absorbing the substances, the shape retention property of the disposable diaper is maintained by the tension of the elastic materials 4 longitudinally traversing the absorbent core 3, thereby leakage of the substances can be prevented.

Next, Figures 9A to 9E are sectional views taken along the lines A-A of Figure 1 to Figure 8A and show various applications of the elastic materials. That is, the rubber strings 4 acting as the elastic materials are attached to the absorbent core 3 of each absorbent product of the first embodiment in such a state that the expansion ratio of the elastic materials 4 increases gradually from the longitudinal centre line to reach maximum at both ends such that the cross section of the absorbent product is curved in an arc shape by the elastic action of the elastic materials 4. Thus, when each absorbent product is applied to the human body, the outer end portions of the absorbent core 3 are more strongly pressed against the skin than the inner portion thereof.

First, in Figure 9A, the liquid pervious inner surface sheet 1, which is composed of the non-woven fabric and positioned on the skin side when applied to the human body, is arranged in a double structure of inner surface sheet 1. Then, the elastic materials 4 shown in Figure 9A are attached within the inner surface sheet 1, such that the expansion ratio thereof gradually increases from the longitudinal centre line toward both ends thereof.

The rubber strings 4 shown in Figure 9B are attached between the inside of the absorbent core 3 and the inner surface sheet 1 such that the expansion ratio thereof gradually increases from the longitudinal centre line toward both ends thereof.

The elastic materials 4 shown in Figure 9C are attached in the absorbent core 3 such that the expansion ratio thereof gradually increases from the longitudinal centre line toward both ends thereof.

The elastic materials 4 shown in Figure 9D are attached between the outside of the absorbent core 3 and the liquid impervious back sheet 2 such that the expansion ratio thereof gradually increases from the longitudinal centre line toward both ends thereof.

Further, in Figure 9E, a non-woven fabric NW is overlapped on the liquid impervious back sheet 2 composed of polyethylene and positioned on the outside when applied to the human body, and the elastic materials 4 shown in Figure 9E are attached between the back sheet 2 and the non-woven fabric NW such that the expansion ratio thereof gradually increases from the longitudinal centre line toward both ends thereof.

As described above, when the elastic materials 4 are attached to the absorbent core 3 by being variously disposed in a state in which the expansion ratio thereof gradually increases from the longitudinal centre line toward both ends and each of the above absorbent products is applied to the human body from the back to the stomach through the crotch, the absorbent core 3, which is interposed between the liquid pervious inner surface sheet 1 facing the skin and the liquid impervious back sheet 2 extending the outer surface of the inner surface sheet 1, is pressed against the skin strongly on the external portions thereof and softly on the internal portion thereof by the difference of the expansion ratios of the elastic materials 4 and is formed in an approximate arc shape having a gap in the inside thereof. Accordingly, when urine, faeces, and body fluids are discharged, even if the weight of the absorbent core 3 is increased by absorbing the substances, the leakage of the substances can be prevented by the difference of the elastic forces of the elastic materials 4.

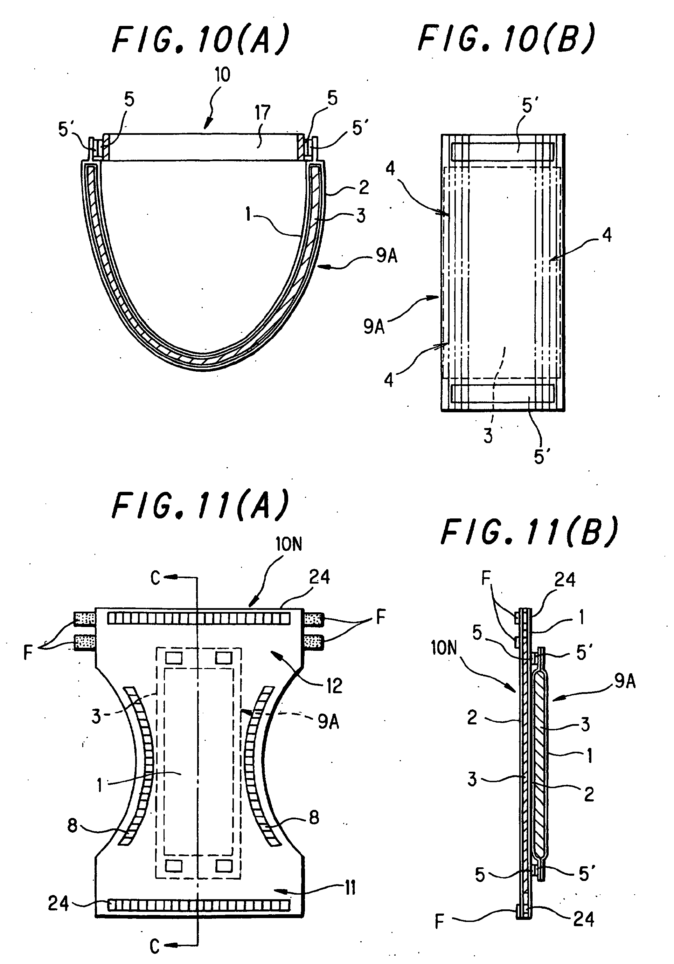

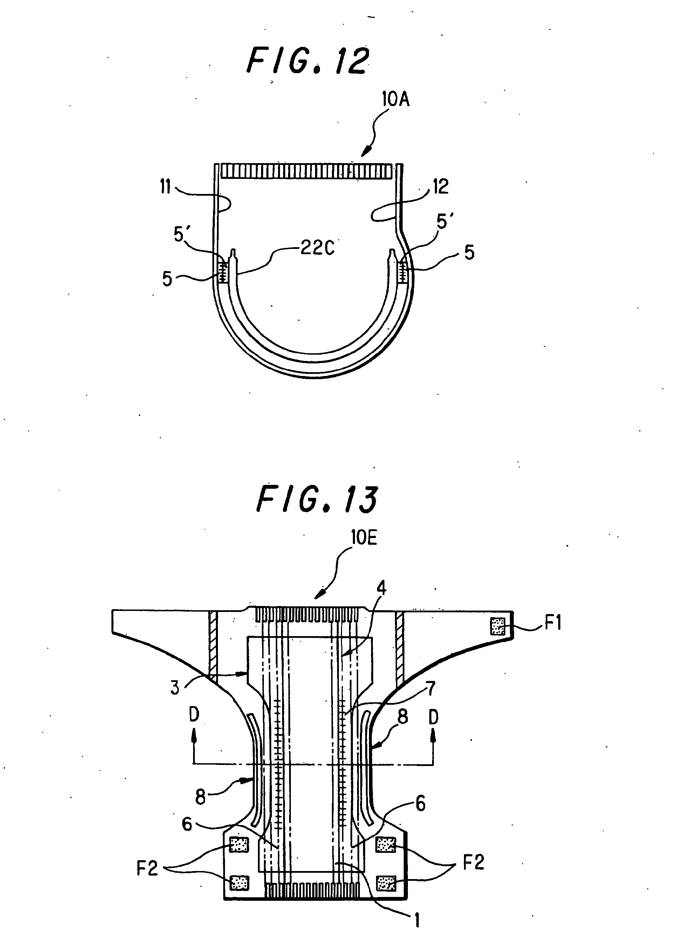

Next, a second embodiment of the present invention will be described with reference to Figures 10 to 13. Figure 10A is a side sectional view of a pad type disposable diaper according to a second embodiment of the present invention having an absorbent product attached to a waist belt holder with a fastening member; Figure 10B is a developed plan view of the absorbent product; Figure 11A is a plan view of a tape type disposable diaper according to the second embodiment of the present invention to which an absorbent product as a pad type disposable diaper is attached; Figure 11B is a sectional view of the tape type disposable diaper taken along the line C-C of Figure 11A, and Figure 12 is a side sectional view of a short type disposable diaper to which a sanitary napkin according to the second embodiment of the present invention is attached.

Reference numeral 17 shown in Figure 10A denotes a waist belt holder. The waist belt holder 17 is formed in an annular shape such that it is attached around the waist of a human body, and fasteners 5 acting as adhering means are attached to the corresponding front and rear outside surfaces of the waist belt holder 17 at two positions. Both ends of the longitudinal absorbent product 9A acting as the absorbent product of the first embodiment shown in Figure 1, for example, are attached to the fasteners 5.

While duplicate explanation is omitted, fasteners 5' are attached to the longitudinal absorbent product 9A at the same intervals as those of the fasteners 5 of the waist belt holder 17 inwardly at both ends thereof in a longitudinal direction as shown in Figure 10B. A pants type disposable diaper 10 is arranged by attaching the fasteners 5' on both sides of, for example, the longitudinal absorbent product 9A to the fasteners 5 of the waist belt holder 17. When the waist belt holder 17 is attached around the waist of a human body, the longitudinal absorbent product 9A attached thereto extends from the back to the stomach through the crotch.

According to this embodiment, the absorbent product 9A is attached to the waist belt holder 17 that is attached around the waist of a human body to extend from the back to the stomach through the crotch. Thus, when urine, faeces, and body fluids are discharged by incontinence and the like, even if the weight of the absorbent core 3 is increased by absorbing the substances, the leakage of the substances can be prevented because the shape retention property of the longitudinal absorbent product 9A is maintained by the tension of the elastic materials 4 longitudinally traversing the absorbent core 3.

Further, reference numeral 10N shown in Figure 11A denotes the tape type disposable diaper including the absorbent product. The tape type disposable diaper 10N is formed of fluffed pulp, synthetic fibres, a super absorbent material, and the like. The tape type disposable diaper 10N is composed of a liquid pervious inner surface sheet 1 positioned on the skin side when the disposable diaper 10N is applied to the human body, a liquid impervious back sheet 2 composed of polyethylene and positioned on the outer surface when the disposable diaper 10N is applied to the human body, and a waist hole section and leg hole sections each including a rectangular absorbent core 3 composed of fluffed pulp and the like and interposed between both sheets 1 and 2.

More specifically, the tape type disposable diaper 10N includes a wide back pad section 12 positioned at the upper end portion so as to face the back, and a wide stomach pad section 11 positioned at the lower end portion extending from the upper end portion so as to face the stomach. A narrow leg section with curved lines is formed between the lower side of the back pad section 12 and the upper side of the stomach pad section 11 so as to connect them to each other. Waist belts or waist elastic materials are longitudinally attached to the back pad section 12 and the stomach pad section 11, and a number of rubber strings 24 acting as elastic materials are disposed along the waist belts or the waist elastic materials from one end of the front to near the square corners, and fasteners F are disposed on both ends of the upper portion. Further, rubber strings 8 are attached to both leg sections along the curved portions thereof between the squared portions.

In the tape type disposable diaper 10N arranged as described above, fasteners 5, 5 are attached at predetermined intervals to the back pad section 12 and the stomach pad section 11 on the skin side of the inner surface sheet 1. The longitudinal absorbent product 9A shown in Figure 10B is attached to these fasteners 5, 5 through the fasteners 5', 5', or non-woven fabrics attached to both ends thereof so as to extend from the back to the stomach through the crotch.

Reference numeral 10A shown in Figure 12 denotes ordinary shorts. Fasteners 5, 5 are longitudinally attached at predetermined intervals to the back pad section 12 and the stomach pad section 11 of the ordinary shorts 10A on the skin side thereof. The sanitary napkin 22C is attached to these fasteners 5, 5 through fasteners 5', 5', or non-woven fabrics attached to the both ends thereof such that the sanitary napkin 22C extends from the back to the stomach through the crotch when applied to the human body.

Thus, according to the second embodiment, since the longitudinal absorbent product 9A, and the like is attached to the waist belt holder 17, the tape type disposable diaper 10N, the shorts 10A, and the like through the fasteners 5', 5' such that the absorbent product 9A and the like extend from the back to the stomach through the crotch when applied to the human body, not only can a secure fit be obtained from the front body around a waist when it is applied to the human body but also the absorbent product 9A can be easily replaced when it becomes dirty.

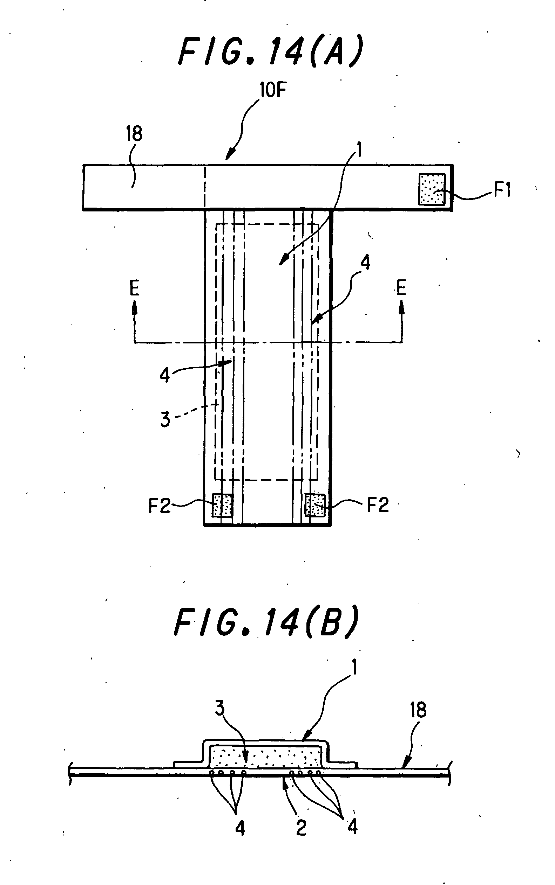

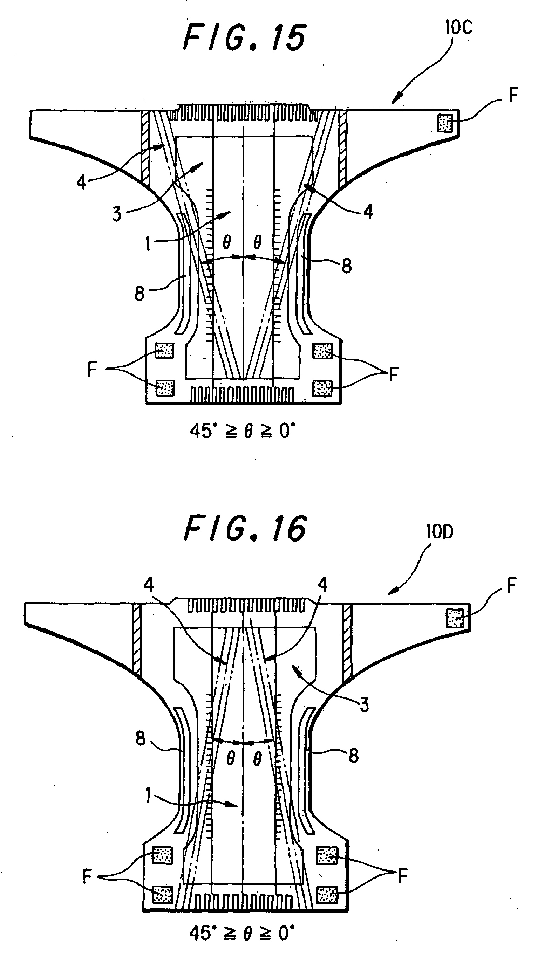

Next, a third embodiment of the present invention will be described with respect to Figures 13 to 18. Figure 13 is a plan view of a wing type disposable diaper according to the third embodiment of the present invention. Figure 14A is a plan view of a T-type disposable diaper. Figure 14B is a sectional view of the T-type disposable diaper taken along the line E-E of Figure 14A. Figure 15 is a plan view of a wing type disposable diaper. Fig. 16 is also a plan view of a wing type disposable diaper.

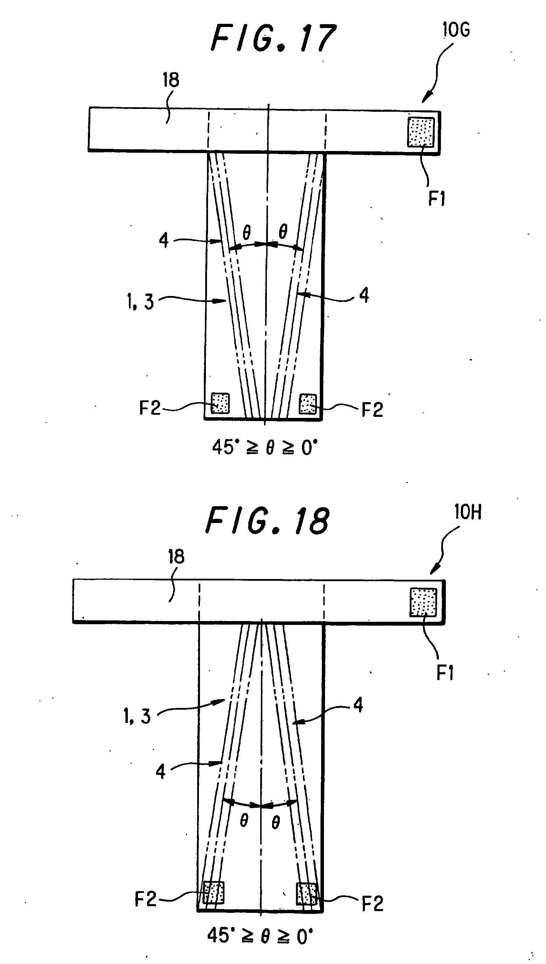

Figure 17 is a plan view of a T-type disposable diaper, and Figure 18 is a plan view of a T-type disposable diaper. Note that the same sections as those of the above sections are denoted by the same reference numerals, and the description thereof is omitted.

Reference numeral 10E shown in Figure 13 denotes the wing type disposable diaper. The wing type disposable diaper 10E includes a laterally long fastening section formed to an upper end portion and having a fastener F1 at one end thereof such that the fastening section is fastened around the stomach. A rectangular absorbent core 3 is interposed between a liquid pervious inner surface sheet 1 and a liquid impervious back sheet 2 in a region extending continuously downward from the lower end of the fastening section so as to be curved and narrowed. Elastic materials 4 are attached to the back sheet 2 from the upper end portion to the lower end portion thereof so as to longitudinally traverse the absorbent core 3, and fasteners F2 are disposed on both end portions of the back sheet 2 at the lower end portion thereof at upper and lower positions.

Rubber strings 8 acting as elastic materials are attached to the back sheet 2 in the narrow parallel leg sections on both sides of the absorbent core 3. Standing cuff gathers, which are composed of standing barrier cuffs 6 and rubber strings 4 that act as elastic materials and are attached to the extreme ends of the barrier cuffs 6, are formed on both sides of the absorbent core 3, thereby the wing type disposable diaper including an absorbent product is arranged.

Thus, according to the wing type disposable diaper including the absorbent product, when the disposable diaper, with the standing cuff gathers having the elastic materials disposed at the extreme ends thereof, is applied to the human body from the back to the stomach through the crotch, the extreme ends of the standing cuff gathers facing the skin are strongly pressed against the skin by an expansion ratio larger than that of the elastic materials 4 of the absorbent core 3. As a result, the rubber strings 7 at the extreme ends of the standing cuff gathers, which are disposed on both sides of the absorbent core 3 when the disposable diaper is applied to the human body, press against the skin more strongly than the elastic force of the inner absorbent core 3. Thus, when urine, faeces, and body fluids are discharged, even if the weight of the absorbent core 3 is increased by absorbing the substances, the leakage of the substances can be prevented by the elastic force of the elastic means 4.

Next, reference numeral 10F shown in Figure 14A denotes the T-type disposable diaper. The T-type disposable diaper 10F has a waist belt 18 disposed to the upper end portion thereof and fastened around the back; a back sheet 2 is longitudinally extended downward from the waist belt 18 so as to extend from the back, where the waist belt 18 is positioned when the disposable diaper is applied to the human body, to the stomach through the crotch, and elastic means 4 acting as string-like elastic materials are longitudinally disposed to the back sheet 2 so as to traverse an absorbent core 3, thereby the T-type disposable diaper 10F including an absorbent product is arranged.

Then, a fastener F1 is attached to either end of the waist belt 18 to attach it around the waist, and fasteners F2 are attached to the lower end of the inner surface sheet 1 on both sides thereof.

Reference numeral 10C shown in Figure 15 denotes the wing type disposable diaper. The wing type disposable diaper 10C includes a laterally long fastening section formed to the upper end portion thereof and having a fastener F1 at one end thereof such that the fastening section is fastened around the stomach. A rectangular absorbent core 3 is interposed between a liquid pervious inner surface sheet 1 and a liquid impervious back sheet 2 in a region extending continuously downward from the lower end of the fastening section so as to be curved and narrowed. Elastic materials 4 are symmetrically attached to the back sheet 2 from the lower end portion to the upper end portion thereof so as to longitudinally traverse the absorbent core 3 in a direction where they spread at an inclining angle of 45° ≥ θ ≥ 0° to the longitudinal centre line of the absorbent core 3.

Then, a quantity of rubber strings 8, which have an elastic expansion ratio larger than that of the elastic means 4, are attached to the back sheet 2 in parallel narrow leg sections on both sides of the absorbent core 3, thereby the wing type disposable diaper including the absorbent product is arranged.

Reference numeral 10D shown in Figure 16 denotes the wing type disposable diaper including the absorbent product. The wing type disposable diaper 10D is the same in arrangement as the wing type disposable diaper 10C except that the elastic materials 4 are symmetrically attached to a back sheet 2 from the upper end portion to the lower end portion thereof so as to longitudinally traverse an absorbent core 3 in a direction where they spread at an inclining angle of 45° ≥ θ ≥ 0° to the longitudinal centre line of the absorbent core 3.

Reference numeral 10G shown in Figure 17 denotes the T-type disposable diaper. The T-type disposable diaper 10G is the same in arrangement as the T-type disposable diaper 10F shown in Figures 14A and 14B except that the elastic materials 4, which are disposed to a back sheet 2 extending from the back to the stomach through the crotch and positioned at the waist 18 when the disposable diaper is applied to the human body, are symmetrically attached in a direction where they spread from the lower end portion to the upper end portion of the back sheet 2 at an inclining angle of 45° ≥ θ ≥ 0°.

Further, reference numeral 10H shown in Figure 18 denotes the T-type disposable diaper. The T-type disposable diaper 10H is the same in arrangement as the T-type disposable diaper 10G except that the elastic means 4 are symmetrically attached to a back sheet 2 in a direction where they spread from the upper end portion to the lower end portion of the back sheet 2 at an inclining angle of 45° ≥ θ ≥ 0°. Note that the expansion ratio of the elastic materials 4 in this embodiment is set to increase from the longitudinal centre line of the absorbent core toward both ends thereof.

According to the third embodiment, when each of the longitudinal disposable diapers including the absorbent product is applied to the human body, the absorbent core 3 facing the skin is held by the elastic materials 4 disposed adjacent to both sides of the absorbent core 3 such that the absorbent core 3 longitudinally traverses the human body from the back to the stomach through the crotch. Therefore, when urine, faeces, and body fluids are discharged, even if the weight of the absorbent core 3 facing the skin is increased by absorbing the substances, the leakage of the substances can be prevented because the formation of a gap in the crotch portion is prevented by the tension of the elastic materials 4 longitudinally traversing the absorbent core 3, and the shape retention property of the longitudinal disposable diaper.

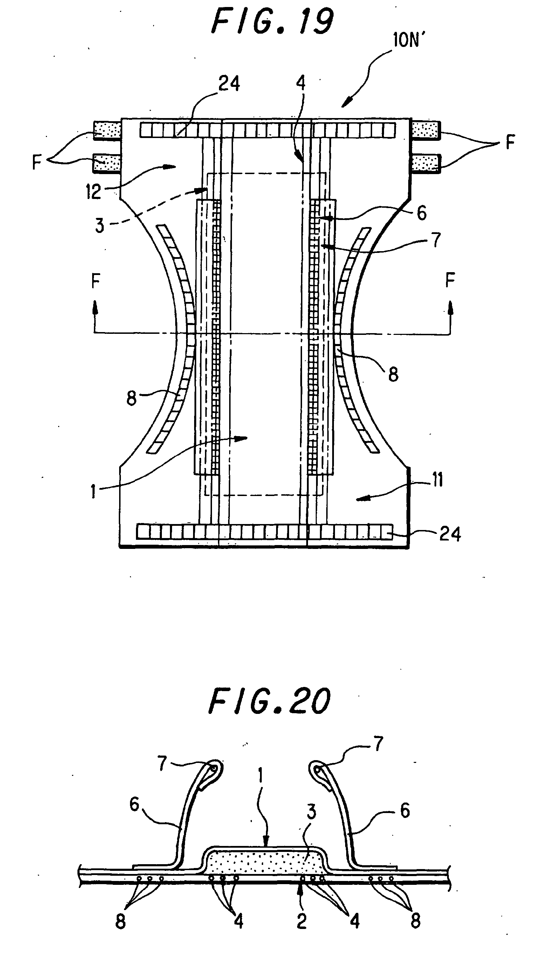

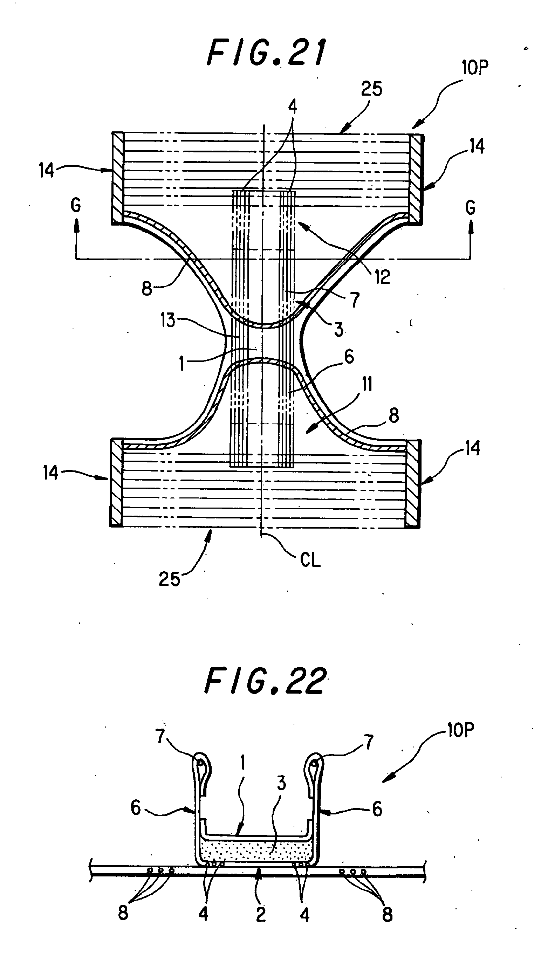

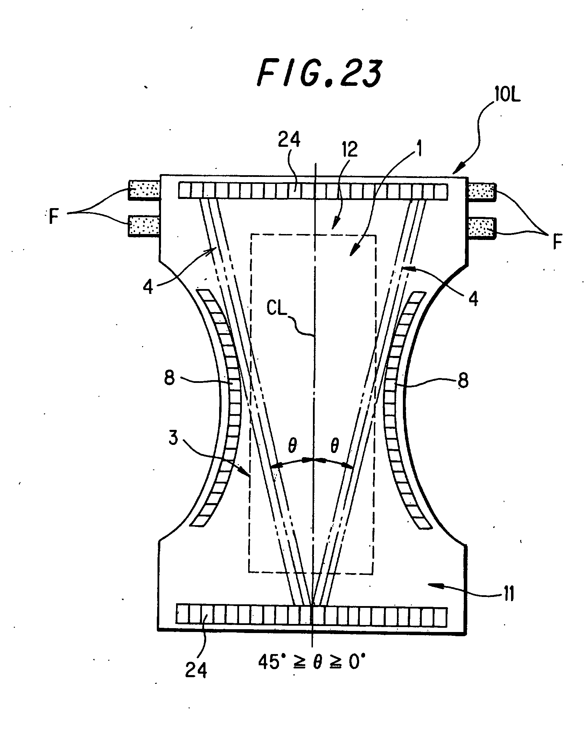

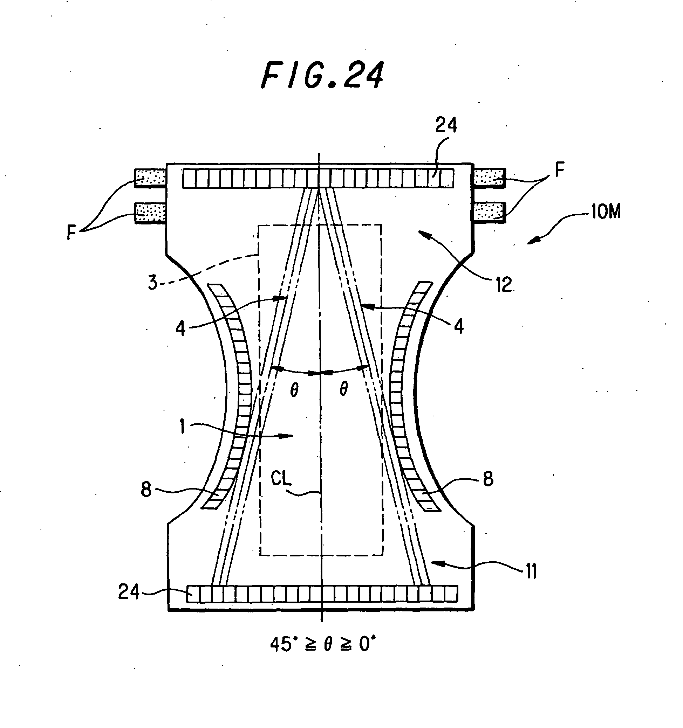

Next, a fourth embodiment of the present invention will be described with reference to Figures 19 to 24. Figure 19 is a plan view of a tape type disposable diaper according to the fourth embodiment of the present invention. Figure 20 is a sectional view of the disposable diaper taken along the line F-F of Figure 19. Figure 21 is a plan view of a pants type disposable diaper according to the fourth embodiment of the present invention. Fig. 22 is a sectional view of the disposable diaper taken along the line G-G of Figure 21. Figure 23 is a plan view of a tape type disposable diaper, and Figure 24 is a plan view of a tape type disposable diaper.

Reference numeral 10N' shown in Figure 19 denotes the tape type disposable diaper. The tape type disposable diaper 10N' is approximately the same in arrangement as the tape type disposable diaper 10N shown in Figure 11A except that standing cuff gathers, which are composed of standing barrier cuffs 6 and rubber strings 7 that act as elastic materials and attached to the extreme ends of the barrier cuffs 6, are formed on both sides of an absorbent core 3 and that a number of elastic materials 4 are directly attached to a back sheet 2 so as to longitudinally traverse the absorbent core 3. Then, the rubber strings 7 of the standing cuff gathers have an expansion ratio larger than that of the elastic materials 4 of the back sheet 2.

Reference numeral 10P shown in Figure 21 denotes the pants type disposable diaper. The pants type disposable diaper 10P is formed of fluffed pulp, synthetic fibres, a super absorbent material, and the like and composed of an inner surface sheet 1, which is formed of a non-woven fabric and positioned on the skin side when the disposable diaper is applied to the human body, a liquid impervious back sheet 2, which is formed of polyethylene and positioned on an outer surface when the disposable diaper is applied to the human body, and a rectangular absorbent core 3 formed of fluffed pulp, and the like and interposed between sheets 1 and 2.

Further, the pants type disposable diaper 10P includes a wide back pad section 12 positioned at the upper end portion thereof so as to face the back, acting as a rear portion, and a wide stomach pad section 11 positioned at the lower end portion thereof extending from the upper end portion so as to face the stomach, acting as a front portion. A narrow leg section having curved lines is formed between both sides of the back pad section 12 and the stomach pad section 11 so as to connect them to each other, and a crotch section 13 is formed as an intermediate portion. Note that reference numeral 14 denotes side seal sections.

The elastic expansion ratio of the rubber strings 7 of the barrier cuffs 6 is set larger than that of elastic materials 4 disposed in parallel with each other on both sides of an absorbent core 3 so as to longitudinally traverse it or disposed adjacent to the back sheet 2.

Thus, when the pants type disposable diaper 10P including the absorbent product fastened to the inner surface sheet 1 is applied to the human body, the absorbent core 10 facing the skin is held by the elastic materials 4 disposed adjacent to both sides of the absorbent core 3 so as to longitudinally traverses a human body from the back to the stomach through the crotch. Therefore, when urine, faeces, and body fluids are discharged, even if the weight of the absorbent core 3 facing the skin is increased by absorbing the substances, the leakage of the substances can be prevented because the formation of a gap in the crotch is prevented by the tension of the elastic materials 4 longitudinally traversing the absorbent core 3 and the shape retention property of the pants type disposable diaper 10P.

Next, reference numeral 10L shown in Figure 23 denotes the tape type disposable diaper. The tape type disposable diaper 10L is fundamentally the same in arrangement as the tape type disposable diaper 10N shown in Figure 11A according to the second embodiment except for the absorbent product and the following point. That is, in the tape type disposable diaper 10L, elastic materials 4 are symmetrically attached to a back sheet 2 so as to longitudinally traverse an absorbent core 3 in a direction where they spread from a lower end portion to an upper end portion at an inclining angle of 45° ≥ θ ≥ 0° to a longitudinal centre line.

Further, reference numeral 10M shown in Figure 24 denotes the tape type disposable diaper. The tape type disposable diaper 10M is approximately the same in arrangement as the tape type disposable diaper 10L shown in Figure 23 except that the elastic materials 4 are symmetrically attached in a direction where they spread from an upper end portion to a lower end portion at an inclining angle of 45° ≥ θ ≥ 0°.

According to the fourth embodiment, when each of the tape type disposable diapers is applied to the human body, the absorbent core 3 facing the skin is held by the elastic materials 4 that are disposed adjacent to both sides of the absorbent core 3 so as to traverse the human body longitudinally from the back to the stomach through the crotch. Thus, when urine, faeces, and body fluids are discharged, even if the weight of the absorbent core 3 facing the skin is increased by absorbing the substances, the leakage of the substances can be prevented because the formation of a gap in the crotch is prevented by the tension of the elastic materials 4 longitudinally traversing the absorbent core 3 and the shape retention property of the tape type disposable diaper.

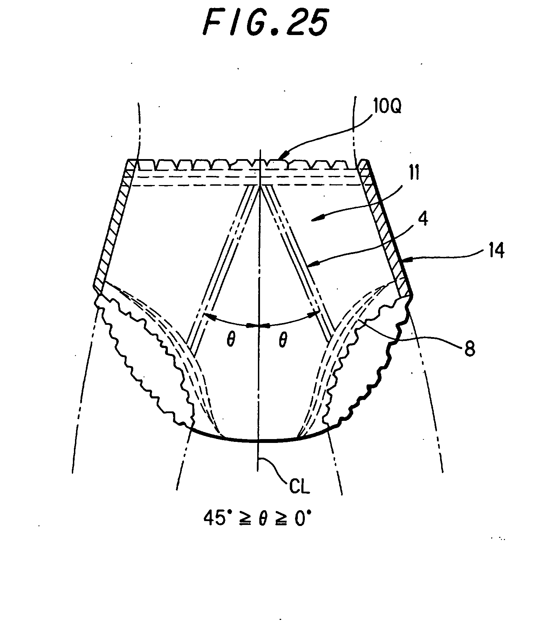

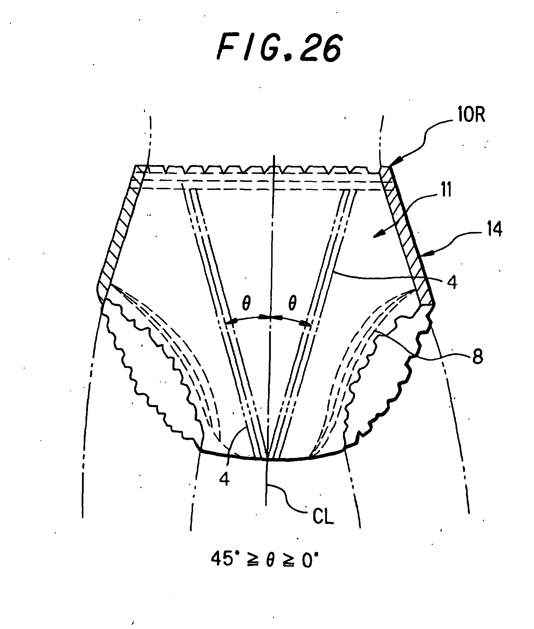

Next, a fifth embodiment of the present invention will be described with reference to Figures 25 and 26. Figure 25 is a plan view of a pants type disposable diaper according to the fifth embodiment of the present invention, and Figure 26 is also a plan view of a pants type disposable diaper according to the fifth embodiment.

Reference numeral 10Q shown in Figure 25 denotes the pants type disposable diaper including the absorbent product. In the pants type disposable diaper 10Q, elastic materials 4 are symmetrically attached to a wide stomach pad portion 11 facing the stomach when the disposable diaper is applied to the human body in a direction where they spread from an upper end portion to a lower end portion at an inclining angle of 45° ≥ θ ≥ 0° to the centre line CL of an absorbent core 3. Then, rubber strings 8 are directly attached to an inner sheet 42 in curved leg sections along both curved portions thereof.

Reference numeral 10R shown in Figure 26 denotes the pants type disposable diaper. The pants type disposable diaper 10R is fundamentally the same in arrangement as the pants type disposable diaper 10Q shown in Figure 25 except that the elastic materials 4 are symmetrically attached in a direction where they spread from the lower end portion to the upper end portion at an inclining angle of 45° ≥ θ ≥ 0°.

According to the fifth embodiment, when each of the pants type disposable diapers is applied to the human body, the elastic means 4 of the absorbent core 3 are symmetrically disposed in a direction where they spread downward from an upper end portion or to the upper end portion from below at an inclining angle of 0° to 45° to the centre line CL of the absorbent core 3. Thus, when urine, faeces, and body fluids are discharged, even if the weight of the absorbent core 3 is increased by absorbing the substances, the absorbent core 3 is stably held by the elastic means 4 and the shape retention property of the disposable diaper can be securely maintained.

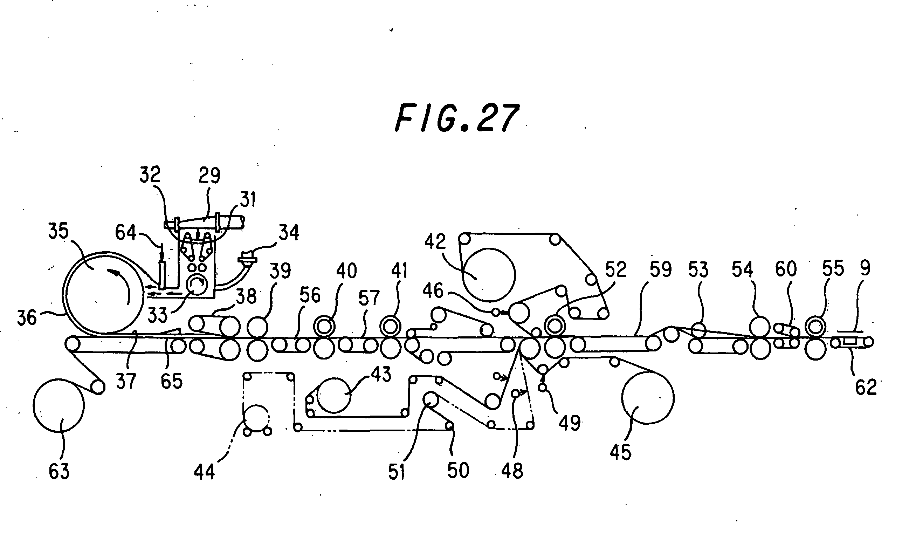

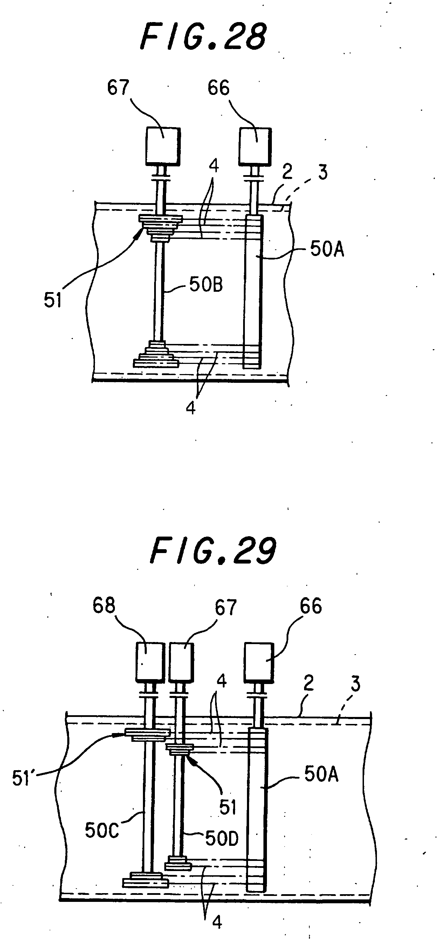

Next, a method of manufacturing an absorbent product according to a sixth embodiment of the present invention will be described with reference to Figures 27 to 29. Figure 27 is a flowchart of a manufacturing process according to a sixth embodiment of the present invention for manufacturing an absorbent product. Figure 28 is a view explaining an example of an apparatus for supplying an elastic means in the state in which tension is applied to an absorbent product such that the expansion ratio thereof sequentially increases to reach maximum at both ends thereof in the manufacturing process of the absorbent product. Figure 29 is a view illustrating another example of the apparatus for supplying the elastic material likewise.

First, the manufacturing process of a pad type disposable diaper as the absorbent product will be described according to a manufacturing flow. As shown in Figure 27, a drum former 35 is an apparatus for uniformly stacking pulp by introducing fluffed pulp (hereinafter, referred to as "fluff pulp") 31, which is a material of an absorbent core and supplied from a supply pipe 29 together with air, to a dispersing roll 33 by feed belts 32 and by blowing the fluff pulp 31 having been dispersed by the dispersing roll 33 onto a mesh wire 36 formed on the surface of a suction drum 35 by air taken from an air intake port 34. In the above process, just before the fluff pulp 31 mixed with the air is blown onto the suction drum 35, a bead-shaped super absorbent material 64 mixed in an air stream is placed on a tissue web where a continuous web of the absorbent core is formed. While the tissue web is transported by a conveyer 37, the tissue web is folded and overlapped on the upper surface thereof by folding both sides thereof by a folding device 65, and then the tissue web is introduced to press rolls 39 through belt presses 38 and pressed by the press rolls 39 such that the tissue web has a predetermined density; thereby a continuous absorbing core sheet 63 is formed.

Subsequently, the absorbing core sheet is transported by a conveyer 56 to improve urine dispersing property and the shape retaining property of the absorbing core sheet 63, and the absorbing core sheet 63 having passed through an emboss roll press 40 is introduced to a conveyer 57 and cut off individually by a mat cutter 41. In the process in which the thus cut-off absorbing core sheets 63 are transported by a conveyer 58, a liquid pervious inner surface sheet 42 is supplied to the upper surfaces of the absorbing core sheets 63, and a liquid impervious back sheet 43 is supplied to the lower surfaces thereof.

A continuous pad type disposable diaper, which is sandwiched between the inner surface sheet 42 and the back sheet 43 by an end side press 52, can be adhered by previously applying binders 46 and 47 such as a hot melt adhesive to the inner surface sheet 42 and the back sheet 43.

The pad type disposable diaper can be continuously manufactured by applying elastic materials 44, a non-woven fabric sheet 45, and the like to the pad type disposable diaper through binders 47 and 48 such as a hot melt adhesive.

The length of the pad type disposable diaper is reduced by the contraction of the elastic means 44 conveyed by a conveyer 62. Then, the pad type disposable diaper is supplied to a tri-folding process while being held by a suction box disposed under the conveyer 62 such that the disposable diaper is not displaced.

The expansion ratio of the elastic materials 44 is regulated by the difference between the surface velocities of elastic material rolls 51 shown in Figure 28 and elastic material rolls 51 and 51' shown in Figure 29. That is, the surface velocities of the elastic material rolls 51 and 51' correspond to the expansion ratio of the elastic materials 44.

Each of the elastic material rolls 51 shown in Figure 28 is arranged as a stepped roll provided with a number of steps such that the roll diameter thereof is increased from the central portion of the absorbent core 3 to the external side edges thereof. The elastic material rolls 51 are disposed at both end portions of a drive shaft 50B driven by a servo motor 67, and the expansion ratio of the elastic materials 44 can be changed coaxially by drawing elastic materials 4 wound between the elastic material rolls 51 and a parallel roller 50A driven by a servo motor 66, respectively.

Next, respective elastic material rolls 51 and 51' shown in Figure 29 are obtained by dividing the stepped rolls 51 shown in Figure 28 and separately disposed to two shafts 50C and 50D driven by servo motors 67 and 68, respectively. The expansion ratio of the elastic means 44 can be changed coaxially by drawing elastic material 44 wound between the elastic material rolls 51 and 51' and the parallel roller 50A driven by the servo motor 66.

That is, the non-woven fabric sheet 45, to which a binder is applied, is positioned on the back sheet 43 that is positioned on the lower surface of the absorbing core sheet 63 together with the elastic materials 44 such that the elastic materials 44 are sandwiched between the back sheet 43 and the non-woven fabric sheet 45 and disposed adjacent to both sides of the absorbing core sheet 63. In this process, the elastic materials 44 are applied with tension such that the expansion ratio of the elastic materials 44 is sequentially increased laterally from the centre to the external portions thereof and attached between the back sheet 43 and the non-woven fabric sheet 45.

With the above arrangement, the elastic materials 44 are attached between the back sheet 43 and the non-woven fabric sheet 45 while being applied with tension, sequentially increasing the expansion ratio of the elastic materials 44 laterally from the centre to the external portions thereof in the process of continuously forming the absorbent product. As a result, the absorbent product, which is curved in an approximate arc shape such that the expansion ratio thereof is sequentially increased laterally from the centre to the external portions thereof can be continuously manufactured effectively in large quantities.

The described embodiments can achieve the following advantages.

As described above in detail, according to the embodiments falling within the scope of claim 1, the elastic materials disposed on the both external portions of the absorbent product are pressed against the skin more strongly as compared with the internal portion thereof when the absorbent product is applied to the human body. Thus, when urine, faeces, body fluids, and the like are discharged, even if the weight of the absorbent core is increased by absorbing the substances, the leakage of the substances can be prevented by the elastic force of the elastic materials.

According to further embodiments, not only can a secure fit be obtained around the waist when the absorbent product is applied to the human body, but it also can be replaced when it becomes dirty because it is fastened through the fastening means.

According to further embodiments, when urine, faeces, and body fluids are discharged, even if the weight of the absorbent core facing the skin is increased by absorbing the substances, the formation of a gap in the crotch portion is prevented by the tension of the elastic materials longitudinally traversing the absorbent core, thereby the leakage of the substances can be prevented because the shape retention property of the wing type and tape type disposable diapers each including the absorbent product is maintained.

According to further embodiments, when urine, faeces, and body fluids are discharged, even if the weight of the absorbent core facing the skin is increased by absorbing the substances, the leakage of the substances can be prevented because the formation of a gap in the crotch portion is prevented by the tension of the elastic materials longitudinally traversing the absorbent core, and the shape retention property of the tape type disposable diaper is maintained.

According to further embodiments, when urine, faeces, and body fluids are discharged, even if the weight of the absorbent core facing the skin is increased by absorbing the substances, the leakage of the substances can be prevented because the formation of a gap in the crotch portion is prevented by the tension of the elastic materials longitudinally traversing the absorbent core and the shape retention property of the pants type disposable diaper is maintained.

According to the embodiments falling within the scope of claim 7, when urine, faeces, body fluids, and the like are discharged, even if the weight of the absorbent core is increased by absorbing the substances, the leakage of the substances can be prevented because the shape retention property of the disposable diaper is maintained by the elastic force of the elastic materials obliquely traversing the absorbent core with respect to the centre line thereof.

According to the embodiments falling within the scope of claim 8, the standing cuff gathers press against the skin more strongly than the elastic absorbent core disposed internally. Thus, when urine, faeces, body fluids, and the like are discharged, even if the weight of the absorbent core is increased by absorbing the substances, the leakage of the substances can be prevented by the large expansion ratio of the elastic materials provided with the standing cuff gathers.

According to the embodiments falling within the scope of claim 9, the elastic materials are attached while being continuously supplied with the tension applied thereto for sequentially increasing the expansion ratio thereof from the centre to both sides laterally in the process of continuously forming the absorbent product. Accordingly, the absorbent product, which is curved in an approximate arc shape such that the expansion ratio thereof sequentially increases laterally from the central portion to both end portions, can be continuously manufactured effectively.

## Claims

1. An absorbent product including:
a liquid pervious inner surface sheet (1);
a liquid impervious back sheet (2) positioned on an outside surface; and
an absorbent core (3) interposed between the inner surface sheet (1) and the back sheet (2),
wherein a plurality of elastic materials (4) are disposed adjacent both sides of the absorbent core (3) so as longitudinally to traverse the absorbent core (3) extending substantially from the back to the stomach through the crotch when the absorbent product is applied to the human body, wherein the expansion ratio of the elastic materials is sequentially increased laterally from the centre to the external portions thereof.

2. A product according to claim 1, wherein the product is provided as a pat type or flat sheet type disposable diaper, as a sanitary napkin or other goods formed of fluffed pulp, synthetic fibres or a super absorbent material.

3. A product according to claim 2, wherein fastening means (5', 5') are attached to both of end portions of the product and the product is fastened to a waist belt holder (17) as well as to tape type diapers (10L, 10M, and 10N), to pants type diapers (10P, 10Q, and 10R) and to diaper extended pants through the fastening materials (5', 5') such that the product extends from the back to the stomach through the crotch when it is applied to the human body.

4. A product according to claim 1, provided as a wing type disposable diaper (IOC, 10D, and 10E), as a T-type disposable diaper (10F, 10G, and 10H) or as a sanitary napkin (10A) provided with either a pants or a belt holder or other goods including the product.

5. A disposable diaper including an absorbent product according to claim 1, wherein the diaper is of a tape type (10L, 10M, 10N) and the product is formed of fluffed pulp, synthetic fibres, a super absorbent material or similar material, the diaper including:
a liquid pervious inner surface sheet (1), positioned on the skin side when the disposable diaper is applied to the human body; and
a liquid impervious back sheet (2) positioned on the outside when the disposable diaper is applied to the human body;
a waist hole section and leg hole sections including an absorbent core interposed between the inner surface sheet (1) and the back sheet (2);
wherein elastic materials (4) are directly attached to the back sheet (2) extending from the back to the stomach through the crotch when the disposable diaper is applied to the human body so as to longitudinally traverse the absorbent core (3).

6. A disposable diaper including a product according to claim 1, wherein the diaper is of a pants type (10P, 10Q, 10R) and the product is formed of fluffed pulp, synthetic fibres, a super absorbent material or similar material; the diaper including:
an outside surface, an inside surface, a front portion, a rear portion and a crotch portion;
a liquid pervious inner surface sheet (1);
a liquid impervious back sheet (2) on the inner surface sheet (42); and
an absorbent core (3) interposed between the inner surface sheet and the back sheet;
wherein elastic materials (4) are directly attached between the inner surface sheet (42) and the back sheet (2), which extends substantially from the back to the stomach through the crotch when the disposable diaper is applied to the human body, and elastic materials (4) are directly attached inside of the inner surface sheet (1), so as longitudinally to traverse the absorbent core.

7. A product according to any of claims 1 to 6, wherein the absorbent core (3) and the elastic materials (4) are substantially symmetrically disposed in a direction where they spread downward from an upper end portion or to the upper end portion from below at an inclining angle of 0° to 45° to the centreline of the absorbent core (3).

8. A product according to any of claims 1 to 7, including standing cuff gathers standing on both sides of the absorbent core (3) and having elastic materials (7, 8) at standing extreme ends thereof, wherein the elastic materials (7, 8) of the standing cuff gathers have an expansion ratio larger than that of the elastic materials (4) of the absorbent core (3).

9. A method of manufacturing an absorbent product of claim 1 including the steps of:
placing a super absorbent material (64) mixed in an air stream on a tissue web where a continuous web is formed just before fluff pulp (31) as a material of an absorbent core is mixed with air and blown onto a suction drum (35);
holding the tissue web on the upper surface thereof by a folding device (65) in a process in which the tissue web is transported by a conveyer;
introducing the tissue web to press rolls (39) through belt presses (38);
forming a continuous absorbent sheet (3) by pressing the tissue web to a predetermined density by the press rolls;
cutting off the absorbent sheet (3) with a mat cutter (41) after passing it through an emboss roll press (40);
joining a liquid pervious inner surface sheet (42) and a liquid impervious back sheet (43) having binders (46, 47) previously applied to one of the upper and lower surfaces thereof to the cut-off absorbent cores; and
positioning a number of string-like elastic means (44) such that the elastic materials are disposed adjacent both sides of the absorbent core in a state in which the elastic materials (44) are applied with tension such that the expansion ratio of the elastic materials (44) is sequentially increased laterally from the centre to the external portions thereof.

## Patentansprüche

1. Absorbierender Artikel umfassend:
eine flüssigkeitsdurchlässige innere Oberflächenfolie (1);
eine flüssigkeitsundurchlässige rückseitige Folie (2), die an einer Außenfläche positioniert ist;
und ein absorbierendes Kernstück (3) zwischen der inneren Oberflächenfolie (1) und der rückseitigen Folie (2),
**wobei** zahlreiche elastische Materialien (4) angrenzend an beide Seiten des absorbierenden Kernstücks (3) so angeordnet sind, dass sie in Längsrichtung entlang des absorbierenden Kernstücks (3) verlaufen, und sich im Wesentlichen vom Rücken zum Bauch durch den Schritt hindurch erstrecken, wenn der absorbierende Artikel an einem menschlichen Körper angebracht ist,
**wobei** sich das Dehnungsvermögen der elastischen Materialien sequentiell von der Mitte zu den seitlichen Außenbereichen hin erhöht.

2. Artikel nach Anspruch 1,
**wobei** der Artikel eine Einwegwindel vom Kissentyp oder flächige Einwegwindel, Damenbinde oder anderer Artikel aus Fluff Pulp, synthetischen Fasern oder einem superabsorbierenden Material ist.

3. Artikel nach Anspruch 2,
**wobei** Befestigungsmittel (5', 5') jeweils an beiden Endbereichen des Artikels angebracht sind und der Artikel sowohl mit einem Hüftgürtelhalter (17) als auch mit Windeln vom Bandtyp (10L, 10M und 10N), an Windeln vom Höschentyp (10P, 10Q und 10R) und an mit windelverstärkten Höschen über die Befestigungsmaterialien (5', 5') befestigt ist, so dass sich der Artikel vom Rücken zum Bauch durch den Schritt hindurch erstreckt, wenn er an den menschlichen Körper angebracht ist.

4. Artikel nach Anspruch 1,
ausgestaltet als Einwegwindel vom Flügeltyp (10C, 10D und 10E), als Einwegwindel vom T-Typ (10F, 10G und 10H) oder als Damenbinde (10A), die entweder mit einem Höschen oder einem Hüfthalter oder einem anderen Produkt einschließlich des Artikels versehen ist.

5. Einwegwindel umfassend einen absorbierenden Artikel nach Anspruch 1,
**wobei** die Windel vom Bandtyp ist (10L, 10M, 10N) und der Artikel aus Fluff Pulp, synthetischen Fasern, einem superabsorbierenden oder ähnlichen Material gebildet ist, wobei die Windel umfasst:
eine flüssigkeitsdurchlässige innere Oberflächenfolie (1), die sich auf der Hautseite befindet, wenn die Einwegwindel an dem menschlichen Körper angebracht ist; und
eine flüssigkeitsundurchlässige rückseitige Folie (2), die sich auf der Außenseite befindet, wenn die Einwegwindel an dem menschlichen Körper angebracht ist;
einen Öffnungsbereich im Bereich der Hüfte und Öffnungsbereiche an den Beinen einschließlich eines absorbierenden Kernstücks, das sich zwischen der inneren Oberflächenfolie (1) und der rückseitigen Folie (2) befindet;
**wobei** elastische Materialien (4) unmittelbar auf der rückseitigen Folie angebracht sind, die sich vom Rücken zum Bauch durch den Schritt hindurch erstreckt, wenn die Einwegwindel an einem menschlichen Körper angebracht ist, und so in Längsrichtung über das absorbierende Kernstück (3) verlaufen.

6. Einwegwindel umfassend einen Artikel nach Anspruch 1,
**wobei** die Windel vom Höschentyp ist (10P, 10Q, 10R) und der Artikel aus Fluff Pulp, synthetischen Fasern, einem superabsorbierenden Material oder einem ähnlichen Material gebildet ist,
**wobei** die Windel umfasst:
eine äußere Oberfläche, eine innere Oberfläche, einen vorderen Bereich, einen Rückenbereich und einen Schrittbereich;
eine flüssigkeitsdurchlässige innere Oberflächenfolie (1),
eine flüssigkeitsundurchlässige rückseitige Folie (2) auf der inneren Oberflächenfolie (42); und ein absorbierendes Kernstück (3) zwischen der inneren Oberflächenfolie und der rückseitigen Folie;
**wobei** elastische Materialien (4) unmittelbar zwischen der inneren Oberflächenfolie (42) und der rückseitigen Folie (2) angebracht sind, die sich im Wesentlichen von der Rückenseite zum Bauch durch den Schritt hindurch erstreckt, wenn die Einwegwindel an einem menschlichen Körper angebracht ist, und die elastischen Materialien (4) unmittelbar auf der Innenseite der inneren Oberflächenfolie (1) angebracht sind, so dass sie in Längsrichtung über das absorbierende Kernstück verlaufen.

7. Artikel nach einem der Ansprüche 1 bis 6,
**wobei** das absorbierende Kernstück (3) und die elastischen Materialien (4) im Wesentlichen symmetrisch in der Richtung angeordnet sind, entlang der sie in einem Neigungswinkel von 0° bis 45° zur Mittellinie des absorbierenden Kernstücks (3) von einem oberen Endbereich nach unten oder von unten zu dem oberen Endbereich hin verlaufen.

8. Artikel nach einem der Ansprüche 1 bis 7,
mit Stehkräuselbündchen, die auf beiden Seiten des absorbierenden Kernstücks 3 verlaufen und elastische Materialien (7, 8) an den abstehenden äußeren Enden aufweisen, wobei die elastischen Materialien (7, 8) der Stehkräuselbündchen ein größeres Dehnungsvermögen als die elastischen Materialien (4) des absorbierenden Kernstücks (3) aufweisen.

9. Verfahren zur Herstellung eines absorbierenden Artikels nach Anspruch 1, umfassend die Schritte:
Aufgeben eines superabsorbierenden Materials (64), das in einem Luftstrom vermischt ist, auf eine Gewebebahn, wobei eine kontinuierliche Bahn geformt wird, unmittelbar bevor Fluff Pulp (31) als Material für das absorbierende Kernstück mit Luft vermischt und auf eine Saugtrommel (35) geblasen wird; halten der Gewebebahn auf deren Oberfläche mit einer Faltvorrichtung (65) während die Gewebebahn mittels eines Förderbands transportiert wird, Zuführen einer Gewebebahn zu Presswalzen (39) über Bandpressen (38),
Formung einer kontinuierlichen absorbierenden Bahn (3), indem die Gewebebahn mittels Presswalzen auf eine gewünschte Dichte verpresst wird,
Zuschneiden der absorbierenden Bahn (3) mit einem Mattenschneider (41) nachdem sie durch eine Prägewalzenpresse (40) geführt worden ist,
Zusammenfügen der zugeschnittenen absorbierenden Kerne mit einer flüssigkeitsdurchlässigen inneren Oberflächenfolie (42) und einer Flüssigkeit undurchlässigen rückseitigen Folie (43), die Bindemittel (46, 47) aufweisen, die zuvor auf eine der Ober- und Unterseiten aufgebracht worden sind, und
Platzieren einer Anzahl von fadenförmigen elastischen Mitteln (44) so, dass die elastischen Materialien benachbart zu beiden Seiten des absorbierenden Kerns in einem Zustand angeordnet sind, indem auf die elastischen Materialen (44) Zug ausgeübt wird, sodass sich das Dehnungsverhältnis der elastischen Materialien (44) sequentiell in seitlicher Richtung von der Mitte zu den Außenbereichen hin erhöht.

## Revendications

1. Produit absorbant incluant :
un feuillet de surface interne perméable aux liquides (1) ;
un feuillet arrière imperméable aux liquides (2) placé sur une surface extérieure ; et
une partie centrale absorbante (3) intercalée entre le feuillet de surface interne (1) et le feuillet arrière (2),
dans lequel une pluralité de matériaux élastiques (4) sont disposés de manière adjacente aux deux côtés de la partie centrale absorbante (3) de façon à traverser longitudinalement la partie centrale absorbante (3) qui s'étend sensiblement depuis le dos jusqu'à l'estomac, en passant par l'entrejambe, lorsque le produit absorbant est appliqué sur le corps humain, dans lequel le rapport de détente des matériaux élastiques augmente séquentiellement, latéralement, entre le centre et les parties extérieures de celui-ci.

2. Produit selon la revendication 1, dans lequel le produit est fourni sous la forme d'une couche jetable du type mini-plaquette ou du type en feuillet plat, sous la forme d'une serviette hygiénique ou d'autres articles composés de matériaux de pâte peluchée, de fibres synthétiques ou d'un matériau super absorbant.

3. Produit selon la revendication 2, dans lequel les moyens d'attache (5', 5') sont fixés sur les deux parties d'extrémité du produit et le produit est fixé sur un support de ceinture pour la taille (17), ainsi que sur des couches du type bande (10L, 10M et 10N), sur des couches du type culotte (10P, 10Q et 10R) et sur des culottes à couches intégrées par l'intermédiaire des moyens d'attache (5', 5'), de telle sorte que le produit s'étend depuis le dos jusqu'à l'estomac en passant par l'entrejambe, lorsqu'il est appliqué sur le corps humain.

4. Produit selon la revendication 1, fourni sous la forme d'une couche jetable du type à oreilles (10C, 10D et 10E) et une couche jetable du type en T (10F, 10G et 10H) ou sous la forme d'une serviette hygiénique (10A) fournie avec soit une culotte soit un support de ceinture et d'autres articles incluant le produit.

5. Couche jetable incluant un produit absorbant selon la revendication 1, dans laquelle la couche est du type bande (10L, 10M, 10N) et le produit est composé d'une pâte peluchée, de fibres synthétiques, d'un matériau super absorbant ou d'un matériau similaire, la couche incluant :
un feuillet de surface interne perméable aux liquides (1) placé du côté de la peau, lorsque la couche jetable est appliquée sur le corps humain ; et
un feuillet arrière imperméable aux liquides (2) placé sur l'extérieur, lorsque la couche jetable est appliquée sur le corps humain ;
une section munie d'un trou pour la taille et des sections munie de trous pour les jambes incluant une partie centrale absorbante intercalée entre le feuillet de surface interne (1) et le feuillet arrière (2) ;
dans laquelle des matériaux élastiques (4) sont directement fixés sur le feuillet arrière (2) qui s'étend depuis le dos jusqu'à l'estomac en passant par l'entrejambe, lorsque la couche jetable est appliquée sur le corps humain, de façon à traverser longitudinalement la partie centrale absorbante (3).

6. Couche jetable incluant un produit selon la revendication 1, dans laquelle la couche est du type culotte (10P, 10Q, 10R) et le produit est composé de pâte peluchée, de fibres synthétiques, d'un matériau super absorbant ou d'un matériau similaire ; la couche incluant:
une surface extérieure, une surface intérieure, une partie avant, une partie arrière et une partie d'entrejambe ;
un feuillet de surface interne perméable aux liquides (1) ;
un feuillet arrière imperméable aux liquides (2) sur le feuillet de surface interne (42) ; et
une partie centrale absorbante (3) intercalée entre le feuillet de surface interne et le feuillet arrière ;
dans laquelle les matériaux élastiques (4) sont directement fixés entre le feuillet de surface interne (42) et le feuillet arrière (2), qui s'étend sensiblement depuis le dos jusqu'à l'estomac en passant par l'entrejambe, lorsque la couche jetable est appliquée sur le corps humain, et les matériaux élastiques (4) sont directement fixés à l'intérieur du feuillet de surface interne (1), afin de traverser longitudinalement la partie centrale absorbante.

7. Produit selon l'une quelconque des revendications 1 à 6, dans lequel la partie centrale absorbante (3) et les matériaux élastiques (4) sont disposés de manière sensiblement symétrique dans une direction où ils s'étalent vers le bas à partir d'une partie d'extrémité supérieure ou jusqu'à une partie d'extrémité supérieure depuis le bas, suivant un angle incliné compris entre 0 et 45 ° par rapport à la ligne centrale de la partie centrale absorbante (3).

8. Produit selon l'une quelconque des revendications 1 à 7, incluant des fronces à revers verticaux qui sont placées sur les deux côtés de la partie centrale absorbante (3) et qui ont des matériaux élastiques (7, 8), au niveau des extrémités verticales de celle-ci, dans lequel les matériaux élastiques (7, 8) des fronces à revers verticaux ont un rapport de détente supérieur à celui des matériaux élastiques (4) de la partie absorbante (3).

9. Procédé de fabrication d'un produit absorbant selon la revendication 1, incluant les étapes consistant à :
placer un matériau super absorbant (64) mélangé dans un flux d'air sur une bande de tissu, lorsqu'une bande continue est formée juste avant que la pâte peluchée (31), en tant que matériau d'une partie centrale absorbante, soit mélangée avec de l'air et soufflée sur un séchoir à tambour aspirant (35) ;
maintenir la bande de tissu sur la surface supérieure de celui-ci grâce à un dispositif de pliage (65) dans un processus dans lequel la bande de tissu est transportée par un convoyeur ;
introduire la bande de tissu dans des rouleaux presseurs (39) à travers des presses à bande (38) ;
former un feuillet absorbant continu (3) en pressant la bande de tissu jusqu'à une densité prédéterminée grâce aux rouleaux presseurs ;
découper le feuillet absorbant (3) avec un couteau à moquette (41) après l'avoir fait passer à travers une presse à rouleau gaufreur (40) ;
assembler un feuillet à surface interne perméable aux liquides (42) et un feuillet arrière imperméable aux liquides (43), des liants (46, 47) ayant été préalablement appliqués sur l'une des surfaces supérieure et inférieure de celui-ci jusqu'aux parties centrales absorbantes découpées ; et
placer un certain nombre de moyens élastiques semblables à des cordons (44), de telle sorte que les matériaux élastiques sont disposés de manière adjacente aux deux côtés de la partie centrale absorbante dans un état dans lequel les matériaux élastiques (44) sont appliqués avec une tension telle que le rapport de détente des matériaux élastiques (44) augmente séquentiellement, latéralement, entre le centre et les parties extérieures de celui-ci.
